# EUROPEAN PATENT APPLICATION

(11) **EP 1 500 927 A1**
(43) Date of publication of application: **26.01.2005**
(21) Application number: 03725679.9
(22) Date of filing: 25.04.2003
(51) Int. Cl.: G01N 27/62, G01N 33/48, G01N 33/68, C07K 14/00

(54) **METHOD OF ANALYZING PROTEIN STRUCTURE, PROTEIN STRUCTURE ANALYZER, PROGRAM AND RECORDING MEDIUM**

(30) Priority: 26.04.2002 JP 2002127399
(71) Applicant: Ajinomoto Co., Inc., Tokyo 104-8315 (JP)
(72) Inventor: YAMADA, Naoyuki, Kawasaki-shi, Kanagawa 210-8681 (JP); SUZUKI, Eiichiro, Kawasaki-shi, Kanagawa 210-8681 (JP); HIRAYAMA, Kazuo, Kawasaki-shi, Kanagawa 210-8681 (JP); UMEYAMA, Hideaki, Urayasu-shi, Chiba 279-0011 (JP)
(74) Representative: Siegert, Georg, Dr.
(86) International application number: PCT/JP2003/005344
(87) International publication number: WO 2003/091720

(57) **Abstract**

This invention fragments ions ionized by an electro-spray ionization method or the like for a target protein whose three-dimensional structure is to be predicted, to fragment ions by a hexapole CID method or the like, and measures a fragmentation spectrum. The present invention determines fragment ion assignment information on an amino acid sequence of the target protein based on the measured fragmentation spectrum. The present invention specifies a region of the amino acid sequence of the target protein in which region the ions are dissociated to the fragment ions according to the determined fragment ion assignment information, and determines easily cleavable domain information on the amino acid sequence of the target protein according to the specified region. The present invention predicts the three-dimensional structure of the target protein. The present invention outputs predicted three-dimensional structure prediction data and the determined easily cleavable domain information while making them correspond to each other.

## Description

### TECHNICAL FIELD

The present invention relates to a protein structure analysis method, a protein structure analyzing instrument, a program and a recording medium. More specifically, the present invention relates to a protein structure analysis method, a protein structure analyzing instrument, a program and a recording medium which can complement three-dimensional structure data on a protein obtained by a calculation scheme by data obtained by a biochemical experiment scheme.

### BACKGROUND ART

Whole genome sequence analyses are performed on many organic species including human, and sequence information thereon is stored in databases (Gerardo Jimenez-Sanchez, Nature 409, 853-855 (2001)). Although a genetic function can be specified and predicted from a genome sequence to some extent, there are many genes whose functions cannot be predicted only from the genome sequence. A protein translated from a DNA sequence of each gene actually fulfils a function of the gene. For a function-unknown gene, a function-known protein that includes highly homologous amino acid sequences is found, thereby predicting the function of the gene. Quite often, however, there are no function-known proteins. In such case, by exploring a protein whose three-dimensional structures is highly analogous or predicting a three-dimensional structure of a protein de novo , then the function of the gene can be predicted.

In order for a protein to exert its function, it is essential that the protein has a three-dimensional structure appropriate to the function. Therefore, homology comparison of the three-dimensional structures gives more accurate prediction of the protein function than homology comparison of amino acid sequences.

As a method for predicting a three-dimensional structure of a protein from an amino acid sequence, whose three-dimensional structure is unknown, a homology modeling method is generally used. This is a calculation-based scientific scheme mainly comprising the following four steps.
(1) When an arbitrary amino acid sequence (target sequence) is given, one or a plurality of proteins (reference proteins) having similar sequences to the target sequence are searched (homology search) from a three-dimensional structure database such as a Protein Data Bank (PDB). An alignment between the target sequence and the similar sequences (aligned sequences) is thereby given.

Computer software for conducting such a database search and alignment includes FASTA, PSI-BLAST, or LIBRA. The FASTA program performs matching of the sequences consisting of 20 kinds of alphabetical characters each of which stands for one of 20 natural amino acids. It is known that a highly reliable model can be constructed if a three-dimensional structure of the reference protein has a high homology with the target (about 30% or more of a degree of match of amino acid that corresponds to about 0.01 or less of an e-value of FASTA).

The PSI-BLAST program performs a similar character sequence matching. However, PSI-BLAST does not calculate information as to whether characters match but calculates a degree of match of characters, that is called profiles, as a substitute matrix of each site on the character sequence of similar proteins, and repeatedly performs the calculation, thereby optimizing an alignment.

The LIBRA program is based on a 3D-1 D method (also referred to as threading method), and searches similar sequences with a known three-dimensional structure as a probe. Therefore, LIBRA obviously differs in search algorithm from FASTA or PSI-BLAST. For this reason, LIBRA is in some cases capable of pointing out a sequence-sequence similarity of a different type from those which can be identified by FASTA and PSI-BLAST.
(2) With the alignment that has been calculated by FASTA, PSI-BLAST, LIBRA, or the like, a correspondence between the target sequence and the similar sequences for each amino acid can be determined. Based on this correspondence, a three-dimensional coordinate of each amino acid on the target sequence is created from three-dimensional coordinates of the reference proteins.
(3) If no counter amino acid is present on the target sequence, an amino acid coordinate at a corresponding position on the reference protein side is not to be used. Conversely, if no counter amino acid is present on the reference protein side, an amino acid coordinate at a corresponding position on the target sequence is created by searching an appropriate coordinate from a protein fragment coordinate database prepared in advance.
(4) In the construction of the protein coordinate through the steps (2) and (3), an inappropriate structural gap, collision or strain may occur between amino acid residues. The structural strain or the like is eliminated by an energy minimization calculation. Some modeling software process the calculation and search procedures at the steps (2) to (4) on all of atoms of the protein not simultaneously but step by step so as to smoothly eliminate the structural strain. Namely, the procedures are performed first on α carbon atoms that build a scaffold of a protein, then on main chain atoms including the α carbon atoms, and finally on all of protein atoms that include side chain atoms.

If an alignment for the target sequence is obtained in the above procedures, the three-dimensional structure of the target sequence can be predicted and constructed.

However, the three-dimensional structure of the protein that is constructed *in silico* by calculation science is not at all experimentally validated. Examples of a method for experimentally examining the three-dimensional structure information of proteins may include an X-ray crystal structure analysis, an electron microscopy, a nuclear magnetic resonance (NMR) and so forth. Whichever method is used, it is technically difficult to obtain the three-dimensional structure of the protein and an operation requiring lots of time and labor has to be carried out.

Recently, a method for obtaining the three-dimensional structure information of the protein using a hydrogen-deuterium (H/D) exchange reaction and mass spectrometry (MS) has been reported. A technique using the H/D exchange reaction is disclosed in, e.g., Patent Application WO00/39326 (Methods for quantifying heavy hydrogen levels at specific peptide amide linkages in protein and peptide) and in a literature (Zhang, Z. and Smith, D. L., (1993) Protein Sci., 2, 522-531.).

With these methods, a protein dissolved in a solution prepared with heavy water is sampled with the lapse of time, the protein is digested in an acid solution using pepsin, and obtained peptides are measured by a LC/MS analysis. A deuteration rate is thereby calculated based on a mass change rate of the peptides. From this result, it is seen that a site at a high deuteration rate has a high solvent accessible ratio or a large flexibility, and that a site at a low deuteration rate has a low solvent accessible ratio.

These methods still have a disadvantage in that it is necessary to execute a large number of sampling procedures and to perform the LC/MS analysis which requires a long measurement time. Further, these methods have disadvantages in that the three-dimensional structure of the protein is greatly changed during pepsin digestion and LC/MS measurement, which elevates tendency of H/D scrambling within inter or intra-peptides. Regarding the latter disadvantage, it is reported that dissociating the protein instead of the pepsin using a capillary skimmer CID method, a fragmentation method using FTICR-MS (Fourier transform ion cyclotron resonance mass spectrometry), gave H/D exchange and information, where a protein-protein interaction may occur (Akashi, S., and Takio, K.: "(2000) Protein Sci, 9,2497-2505, and Akashi Satoko: "Abstracts of The 48th Annual Conference on Mass Spectrometry", p. 208 (2000)). However, even if this method is used, the disadvantage of the need to execute a large number of sampling procedures remains unsolved.

Further, it has been reported by Yamada et al. that protein-protein interactive site information was obtained by a method that was a combination of a fragmentation method using the FTICR-MS, the H/D exchange method, and affinity chromatography (Yamada N., Suzuki E., Hirayama K., (2002) Rapid Commun. Mass Spectrom., 16, 293-299.). With this method, the interactive site information can be obtained with less number of sampling procedures.

As explained above, in the conventional three-dimensional structure prediction of the protein, the three-dimensional structure is constructed based on the amino acid sequence using a calculator. However, the structure is merely constructed virtually and not validated experimentally. Therefore, in order to compare the predicted structure with an actual three-dimensional structure and to evaluate the predicted structure, an X-ray crystal structure analysis or a structure analysis using the NMR or the like should be performed. In order to perform such analyses, a large quantities of proteins, crystallization, stable isotope labeling and the like are required. Consequently, it disadvantageously requires considerable labor and time.

The method using a combination of the MS and the H/D exchange reaction gives the protein three-dimensional structure information with high sensitivity but with low accuracy. However, the method also disadvantageously takes labor and time for experiments.

It is, therefore, an object of the present invention to provide a three-dimensional structure information measurement technique that enables an evaluation of a three-dimensional structure prediction result, that provides structure information useful for structure prediction, and that ensures a high sensitivity and promptness.

### DISCLOSURE OF THE INVENTION

To solve the aforementioned disadvantages, the inventors of the present invention studied a fragmentation technique in the mass spectrometry (MS) and resulting structure information obtained thereby in detail to develop a method for obtaining protein three-dimensional structure information promptly with high sensitivity. As a result, it was found out that the conventional method using the H/D exchange reaction still has disadvantages such as the need of a large number of sampling procedures and the occurrence of the H/D scrambling.

The inventors then studied measurement methods such as a hexapole assisted capillary skimmer CID method (hereinafter, "hexapole CID method") and an infrared multiphoton dissociation method (hereinafter "IRMPD method"), and a resulting fragmentation spectrum was obtained by each method. As a result, the inventors discovered that fragmentation reflecting a secondary structure, a tertiary structure, a flexibility, and the like of a protein occurred.

That is, it was discovered that, in a protein structure, the fragmentation occurs less frequently on a rigid site that has the secondary structure such as an α helix or a β strand, and the fragmentation occurs more frequently on a high motility site such as a loop.

Moreover, by comparing an amino acid sequence of a protein whose three-dimensional structure is desired to be found out with the secondary structures of candidate proteins whose three-dimensional structures are known and whose ranking is given using the 3D-1D method, a number of the candidate protein(s) to be used for the three-dimensional structure prediction can be narrowed down.

Furthermore, by making reference to the structure predicted by the calculation scheme for protein fragmentation data obtained by MS, the predicted structure can be evaluated.

The present invention has been finally completed based on the above knowledge.

To accomplish the object, a protein structure analysis method, a protein structure analyzing instrument, and a program according to one aspect of the present invention comprise a fragmentation spectrum measuring step (a fragmentation spectrum measuring unit) of dissociating a target protein whose three-dimensional structure is to be predicted, and of measuring a fragmentation spectrum thereof; an assignment information determining step (an assignment information determining unit) of determining fragment ion assignment information on an amino acid sequence of the target protein based on the fragmentation spectrum measured at the fragmentation spectrum measuring step (by the fragmentation spectrum measuring unit); an easily cleavable domain information determining step (an easily cleavable domain information determining unit) of specifying a region of the amino acid sequence of the target protein, in which said region is dissociated to fragment ions, using the fragment ion assignment information determined at the assignment information determining step (by the assignment information determining unit), and of determining easily cleavable domain information on the amino acid sequence of the target protein according to the specified region; a three-dimensional structure predicting step (a three-dimensional structure predicting unit) of predicting the three-dimensional structure of the target protein; and a processing result output step (a processing result output unit) of outputting three-dimensional structure prediction data predicted at the three-dimensional structure predicting step (by the three-dimensional structure predicting unit) and the easily cleavable domain information determined at the easily cleavable domain information determining step (by the easily cleavable domain information determining unit) while making them correspond to each other.

According to the method, the instrument, and the program, the target protein whose three-dimensional structure is to be predicted is ionized by an electro-spray ionization method or the like. The resulting ions are dissociated to fragment ions by the hexapole CID method or the like, and the fragmentation spectrum is measured. The fragment ion assignment information on an amino acid sequence of the target protein is determined based on the measured fragmentation spectrum. A region of the amino acid sequence of the target protein, in which said region is dissociated to the fragment ions, is specified according to the determined fragment ion assignment information, and easily cleavable domain information on the amino acid sequence of the target protein is determined according to the specified region. The three-dimensional structure of the target protein is thus predicted. Predicted three-dimensional structure prediction data and the determined easily cleavable domain information are output (e.g., by graphic display or display of a list using a table) while making them correspond to each other. Upon predicting the three-dimensional structure of a protein (including a gene that codes the protein) whose three-dimensional structure is unknown, a highly accurate prediction can be performed by the aforementioned features since the measured three-dimensional structure information can be thereby taken into consideration.

A protein structure analysis method, a protein structure analyzing instrument, and a program according to another aspect of the present invention are characterized in that, at the processing result output step (by the processing result output unit) in the aforementioned protein structure analysis method, protein structure analyzing instrument and program, the three-dimensional structure prediction data is graphically displayed as any one of a wire model, a ribbon model, a pipe model, a ball-and-stick model and a space filling model, and displayed in association with the easily cleavable domain information with a corresponding displayed region of the three-dimensional structure prediction data.

This shows one example of the processing result output more specifically. According to the method, the instrument, and the program, the three-dimensional structure prediction data is graphically displayed as any one of a wire model, a ribbon model, a pipe model, a ball-and-stick model and a space filling model, and displayed in association with the easily cleavable domain information with a corresponding displayed region of the three-dimensional structure prediction data (e.g., by link setting, display on the model, display with a specific pattern such as a shading pattern or a slant-line pattern corresponding to the easily cleavable domain information on the model). Therefore, these pieces of information can be visually and recognizably displayed, and the user can intuitively determine the reliability of the three-dimensional structure prediction data.

A protein structure analysis method, a protein structure analyzing instrument, and a program according to still another aspect of the present invention are characterized in that, at the processing result output step (by the processing result output unit) in the aforementioned protein structure analysis method, protein structure analyzing instrument and program, the three-dimensional structure prediction data is displayed in a different color according to its corresponding easily cleavable domain information.

This shows one example of the processing result output more specifically. According to the method, the instrument, and the program, when the three-dimensional structure prediction data is displayed by graphically or using a table, the three-dimensional structure prediction data is displayed in a different color according to its corresponding easily cleavable domain information. Therefore, these pieces of information can be visually and recognizably displayed, and the user can intuitively determine the reliability of the three-dimensional structure prediction data.

A protein structure analysis method, a protein structure analyzing instrument and a program according to still another aspect of the present invention further comprise a predicted structure evaluation information determining step (a predicted structure evaluation information determining unit) of comparing the three-dimensional structure prediction data predicted at the three-dimensional structure predicting step (by the three-dimensional structure predicting unit) with the easily cleavable domain information determined at the easily cleavable domain information determining step (by the easily cleavable domain information determining unit), of evaluating a predicted structure region corresponding to an easily cleavable domain specified by the easily cleavable domain information, and of determining predicted structure evaluation information; and a predicted structure evaluation information output step (a predicted structure evaluation information output unit) of outputting the predicted structure evaluation information determined at the predicted structure evaluation information determining step (by the predicted structure evaluation information determining unit).

According to the method, the instrument, and the program, the predicted three-dimensional structure prediction data is compared with the determined easily cleavable domain information, a predicted structure region corresponding to an easily cleavable domain specified by the easily cleavable domain information is evaluated, predicted structure evaluation information is determined thereby, and the determined predicted structure evaluation information is output. Therefore, the predicted structure predicted by the calculator can be evaluated based on any biochemical experiment data, and prediction accuracy can be remarkably improved.

This feature also makes it possible to remarkably improve its accuracy for predicting a function based on the three-dimensional structure in case the functional analysis is to be carried for the interesting gene or protein that is found in a genomic sequence analysis, an expression profiling analysis using a DNA chip, a proteome analysis or the like. In case of the genomic sequence analysis, the function of interest could be more efficiently predicted by performing functional analysis based on the three-dimensional structure of the function-unknown gene or protein. Besides, this feature makes it possible to efficiently perform drug design of, e.g., an inhibitor, improvement of activity based on protein engineering, and design of a functionally modified substance.

A protein structure analysis method, a protein structure analyzing instrument and a program according to still another aspect of the present invention further comprise a normal vibration calculating step (a normal vibration calculating unit) of calculating a normal vibration for the target protein, wherein at the processing result output step (by the processing result output unit), a calculation result obtained at the normal vibration calculating step (by the normal vibration calculating unit) is displayed in association with the corresponding displayed region of the three-dimensional structure prediction data.

According to the method, the instrument and the program, the normal vibration is calculated for the target protein, and the normal vibration-mode analysis result (e.g., flexibility information) is displayed in association with the corresponding displayed region of the three-dimensional structure prediction data (e.g., displayed as a vector on the three-dimensional structure model). Therefore, the flexibility information can be considered as the easily cleavable domain. Namely, by obtaining the flexibility information based on the normal vibration-mode analysis result and comparing the flexibility information with the MS measurement data, the prediction model can be evaluated.

A protein structure analysis method, a protein structure analyzing instrument and a program according to still another aspect of the present invention are characterized in that, at the fragmentation spectrum measuring step (by the fragmentation spectrum measuring unit) in the aforementioned protein structure analysis method, protein structure analyzing instrument and program, the target protein is dissociated to the fragment ions by at least one method of collisionally activated dissociation or collision induced dissociation (CID) methods that include a hexapole CID method, a nozzle skimmer CID method, a capillary skimmer CID method, an SORI-CID method and a multi-pole store assisted capillary skimmer CID method, an IRMPD method, an in-source decay (ISD) method, a post-source decay (PSD) method, a surface-induced dissociation (SID) method, an electron capture dissociation (ECD) method and a black-body infrared radiative dissociation (BIRD) method, and the fragmentation spectrum is measured.

This shows one example of the fragmentation spectrum measurement more specifically. According to the method, the instrument and the program, the target protein is dissociated to the fragment ions by at least one method of collisionally activated dissociation or collision induced dissociation (CID) methods that include a hexapole CID method, a nozzle skimmer CID method, a capillary skimmer CID method, an SORI-CID method and a multi-pole store assisted capillary skimmer CID method, an IRMPD method, an in-source decay (ISD) method, a post-source decay (PSD) method, a surface-induced dissociation (SID) method, an ECD method and a BIRD method, and the fragmentation spectrum is measured. Therefore, the fragment ions that reflect the three-dimensional structure of the target protein can be efficiently generated.

A protein structure analysis method, a protein structure analyzing instrument and a program according to still another aspect of the present invention are characterized in that, at the fragmentation spectrum measuring step (by the fragmentation spectrum measuring unit) in the aforementioned protein structure analysis method, protein structure analyzing instrument and program, the target protein is dissociated by proteolysis with enzymatic activity, and the fragmentation spectrum is thereby measured.

This shows one example of the fragmentation spectrum measurement more specifically. According to the method, the instrument and the program, the target protein is fragmented by proteolysis with enzymatic activity to the fragmented peptides, and the mass spectrum and fragmentation spectrum of the fragmented peptides are thereby measured. Therefore, the fragmentation that reflects the three-dimensional structure of the target protein can be efficiently performed.

A protein structure analysis method, a protein structure analyzing instrument and a program according to still another aspect of the present invention comprise a fragmentation spectrum measuring step (a fragmentation spectrum measuring unit) of dissociating a target protein whose three-dimensional structure is to be predicted, and of measuring a fragmentation spectrum thereof; an assignment information determining step (an assignment information determining unit) of determining fragment ion assignment information on an amino acid sequence of the target protein based on the fragmentation spectrum measured at the fragmentation spectrum measuring step (by the fragmentation spectrum measuring unit); a solvent accessible residue information determining step (a solvent accessible residue information determining unit) of specifying a region of the amino acid sequence of the target protein, in which said region is in contact with a solvent, based on the fragmentation spectrum measured at the fragmentation spectrum measuring step (by the fragmentation spectrum measuring unit) and the fragment ion assignment information determined at the assignment information determining step (by the assignment information determining unit), and of determining solvent accessible residue information on the amino acid sequence of the target protein according to the specified region; a three-dimensional structure predicting step (a three-dimensional structure predicting unit) of predicting the three-dimensional structure of the target protein; and a processing result output step (a processing result output unit) of outputting three-dimensional structure prediction data predicted at the three-dimensional structure predicting step (by the three-dimensional structure predicting unit) and the solvent accessible residue information determined at the solvent accessible residue information determining step (by the solvent accessible residue information determining unit) while making them correspond to each other.

According to the method, the instrument and the program, the target protein, whose three-dimensional structure is to be predicted, is dissociated, and the fragmentation spectrum is measured. The fragment ion assignment information on an amino acid sequence of the target protein is determined based on the measured fragmentation spectrum. A region of the amino acid sequence of the target protein, in which said region is in contact with a solvent, is specified according to the measured fragmentation spectrum and the determined fragment ion assignment information, and solvent accessible residue information on the amino acid sequence of the target protein is determined according to the specified region. The three-dimensional structure of the target protein is then predicted. Predicted three-dimensional structure prediction data and the determined solvent accessible residue information are output (e.g., by graphic display or display of a list using a table) while making them correspond to each other. Therefore, upon predicting the three-dimensional structure of a protein (including a gene that codes the protein) whose three-dimensional structure is unknown, a highly accurate prediction can be performed by adding the actually measured three-dimensional structure information.

A protein structure analysis method, a protein structure analyzing instrument and a program according to still another aspect of the present invention are characterized in that, at the processing result output step (by the processing result output unit) in the aforementioned protein structure analysis method, protein structure analyzing instrument and program, the three-dimensional structure prediction data is graphically displayed as any one of a wire model, a ribbon model, a pipe model, a ball-and-stick model and a space filling model, and further characterized in that the solvent accessible residue information is displayed in association with a corresponding displayed region of the three-dimensional structure prediction data.

This shows one example of the processing result output more specifically. According to the method, the instrument, and the program, the three-dimensional structure prediction data is graphically displayed as any one of a wire model, a ribbon model, a pipe model, a ball-and-stick model and a space filling model, and the solvent accessible residue information is displayed in association with a corresponding displayed region of the three-dimensional structure prediction data (e.g., by link setting, display on the model, display with a specific pattern such as a shading pattern or a slant-line pattern corresponding to the solvent accessible domain information on the model). Therefore, these pieces of information can be visually and recognizably displayed, and the user can intuitively determine the reliability of the three-dimensional structure prediction data.

A protein structure analysis method, a protein structure analyzing instrument and a program according to still another aspect of the present invention are characterized in that, at the processing result output step (by the processing result output unit) in the aforementioned protein structure analysis method, protein structure analyzing instrument and program, the three-dimensional structure prediction data is displayed in a different color according to its corresponding solvent accessible residue information.

This shows one example of the processing result output more specifically. According to the method, the instrument and the program, when the three-dimensional structure prediction data is displayed graphically or using a table, the three-dimensional structure prediction data is displayed in a different color according to its corresponding solvent accessible residue information. Therefore, these pieces of information can be visually and recognizably displayed, and the user can intuitively determine the reliability of the three-dimensional structure prediction data.

A protein structure analysis method, a protein structure analyzing instrument and a program according to still another aspect of the present invention further comprise a predicted structure evaluation information determining step (a predicted structure evaluation information determining unit) of comparing the three-dimensional structure prediction data predicted at the three-dimensional structure predicting step (by the three-dimensional structure predicting unit) with the solvent accessible residue information determined at the solvent accessible residue information determining step (by the solvent accessible residue information determining unit), of evaluating a predicted structure region corresponding to a solvent accessible residue specified by the solvent accessible residue information, and of determining predicted structure evaluation information; and a predicted structure evaluation information output step (a predicted structure evaluation information output unit) of outputting the predicted structure evaluation information determined at the predicted structure evaluation information determining step (by the predicted structure evaluation information determining unit).

According to the method, the instrument and the program, the predicted three-dimensional structure prediction data is compared with the determined solvent accessible residue information, a predicted structure region corresponding to the specified solvent accessible residue by the solvent accessible residue information is evaluated; predicted structure evaluation information is determined; and the determined predicted structure evaluation information is then output. Therefore, the predicted structure predicted by the calculator can be evaluated based on any biochemical experiment data, and prediction accuracy can be remarkably improved.

Further, this feature makes it possible to remarkably improve its accuracy for predicting a function based on the three-dimensional structure in case functional analysis is to be carried for the interesting gene or protein that is found in a genomic sequence analysis, an expression profiling analysis using a DNA chip, a proteome analysis or the like. In case of the genomic sequence analysis, the function of interest could be more efficiently predicted by performing functional analysis based on the three-dimensional structure of the function-unknown gene or protein. Besides, this feature makes it possible to efficiently perform drug design of, e.g., an inhibitor, improvement of activity based on protein engineering, and design of a functionally modified substance.

A protein structure analysis method, a protein structure analyzing instrument and a program according to still another aspect of the present invention further comprise a normal vibration-mode analysis step (a normal vibration-mode analysis unit) of calculating a normal vibration for the target protein, wherein at the processing result output step (by the processing result output unit), a calculation result obtained at the normal vibration calculating step (by the normal vibration calculating unit) is displayed in association with the corresponding displayed region of the three-dimensional structure prediction data.

According to the method, the instrument, and the program, the normal vibration is calculated for the target protein, and the normal vibration-mode analysis result (e.g., flexibility information) is displayed in association with the corresponding displayed region of the three-dimensional structure prediction data (e.g., displayed as a vector on the three-dimensional structure model). Therefore, the flexibility information can be considered as the solvent accessible domain. Namely, by obtaining the flexibility information based on the normal vibration-mode analysis result and comparing the flexibility information with the MS measurement data, the prediction model can be evaluated.

A protein structure analysis method, a protein structure analyzing instrument and a program according to still another aspect of the present invention are characterized in that, in the aforementioned protein structure analysis method, protein structure analyzing instrument and program, at the fragmentation spectrum measuring step (by the fragmentation spectrum measuring unit), the target protein is dissociated after a hydrogen-deuterium exchange reaction is provoked for the target protein, and the fragmentation spectrum is thereby measured; and at the solvent accessible residue information determining step (by the solvent accessible residue information determining unit), a deuteration rate of each amino acid residue in the amino acid sequence of the target protein is determined according to the fragmentation spectrum measured at the fragmentation spectrum measuring step (by the fragmentation spectrum measuring unit) and the fragment ion assignment information determined at the assignment information determining step (by the assignment information determining unit), a region of the amino acid sequence of the target protein, in which said region is in contact with a solvent, is specified according to the deuteration rate, and solvent accessible residue information on the amino acid sequence of the target protein is determined according to the specified region.

This shows one example of the fragmentation spectrum measurement more specifically. According to the method, the instrument and the program, the target protein is dissociated after a hydrogen-deuterium exchange reaction is provoked for the target protein; the fragmentation spectrum is thereby measured; a deuteration rate of each amino acid residue in the amino acid sequence of the target protein is determined according to the measured fragmentation spectrum and the determined fragment ion assignment information; a region of the amino acid sequence of the target protein in which said region is in contact with a solvent is specified according to the deuteration rate; and solvent accessible residue information on the amino acid sequence of the target protein is determined according to the specified region. Therefore, the solvent accessible information can be efficiently obtained.

A protein structure analysis method, a protein structure analyzing instrument and a program according to still another aspect of the present invention are characterized in that, in the aforementioned protein structure analysis method, protein structure analyzing instrument and program, at the fragmentation spectrum measuring step (by the fragmentation spectrum measuring unit), the target protein is dissociated after a chemical modification is provoked for the target protein, and the fragmentation spectrum is thereby measured; and at the solvent accessible residue information determining step (by the solvent accessible residue information determining unit), a chemically modified region of each amino acid residue in the amino acid sequence of the target protein is determined according to the fragmentation spectrum measured at the fragmentation spectrum measuring step (by the fragmentation spectrum measuring unit) and the fragment ion assignment information determined at the assignment information determining step (by the assignment information determining unit); a region of the amino acid sequence of the target protein, in which said region is in contact with a solvent, is specified according to the chemically modified region; and solvent accessible residue information on the amino acid sequence of the target protein is determined according to the specified region.

This shows one example of the fragmentation spectrum measurement more specifically. According to the method, the instrument and the program, the target protein is dissociated after a chemical modification is provoked for the target protein; the fragmentation spectrum is thereby measured; a chemically modified region of each amino acid residue in the amino acid sequence of the target protein is determined according to the measured fragmentation spectrum and the determined fragment ion assignment information; a region of the amino acid sequence of the target protein, which region is in contact with a solvent, is specified according to the chemically modified region; and solvent accessible residue information on the amino acid sequence of the target protein is determined according to the specified region. Therefore, the solvent accessible information can be efficiently obtained.

A protein structure analysis method, a protein structure analyzing instrument, and a program according to still another aspect of the present invention are characterized in that, at the fragmentation spectrum measuring step (by the fragmentation spectrum measuring unit) in the aforementioned protein structure analysis method, protein structure analyzing instrument and program, the target protein is dissociated to the fragment ions by at least one method of collisionally activated dissociation or collision induced dissociation (CID) methods that include a hexapole CID method, a nozzle skimmer CID method, a capillary skimmer CID method, an SORI-CID method and a multi-pole store assisted capillary skimmer CID method, an IRMPD method, an in-source decay (ISD) method, a post-source decay (PSD) method, a surface-induced dissociation (SID) method, an ECD method and a BIRD method, and the fragmentation spectrum is measured.

This shows one example of the fragmentation spectrum measurement more specifically. According to the method, the instrument, and the program, the target protein is dissociated to the fragment ions by at least one method of collisionally activated dissociation or collision induced dissociation (CID) methods that include a hexapole CID method, a nozzle skimmer CID method, a capillary skimmer CID method, an SORI-CID method and a multi-pole store assisted capillary skimmer CID method, an IRMPD method, an in-source decay (ISD) method, a post-source decay (PSD) method, a surface-induced dissociation (SID) method, an ECD method and a BIRD method, and the fragmentation spectrum is measured. Therefore, the fragment ions that reflect the three-dimensional structure of the target protein can be efficiently generated.

A protein structure analysis method, a protein structure analyzing instrument, and a program according to still another aspect of the present invention are characterized in that, at the fragmentation spectrum measuring step (by the fragmentation spectrum measuring unit) in the aforementioned protein structure analysis method, protein structure analyzing instrument and program, the target protein is fragmented by proteolysis with enzymatic activity to the fragment peptides, and the mass spectrum and the fragmentation spectrum of the fragmented peptides are measured.

This shows one example of the fragmentation spectrum measurement more specifically. According to the method, the instrument, and the program, by dissociating the target protein by an enzymatic reaction, and further combining the H/D exchange method or the chemical modification method, the solvent accessible site information or the like can be efficiently obtained.

A protein structure analysis method, a protein structure analyzing instrument and a program according to still another aspect of the present invention comprise a fragmentation spectrum measuring step (a fragmentation spectrum measuring unit) of dissociating a complex of a target protein whose three-dimensional structure is to be predicted and a compound, and of measuring a fragmentation spectrum thereof; an assignment information determining step (an assignment information determining unit) of determining fragment ion assignment information on both of or one of the target protein and the compound based on the fragmentation spectrum measured at the fragmentation spectrum measuring step (by the fragmentation spectrum measuring unit); an contact region information determining step (an contact region information determining unit) of determining contact region information in a protein complex on both of or one of the target protein and the compound according to the fragmentation spectrum measured at the fragmentation spectrum measuring step (by the fragmentation spectrum measuring unit) and the fragment ion assignment information determined at the assignment information determining step (by the assignment information determining unit); a three-dimensional structure predicting step (a three-dimensional structure predicting unit) of predicting the three-dimensional structure of both of or one of the target protein and the compound; and a processing result output step (a processing result output unit) of outputting three-dimensional structure prediction data predicted at the three-dimensional structure predicting step (by the three-dimensional structure predicting unit) and the contact region information determined at the contact region information determining step (by the contact region information determining unit) while making them correspond to each other.

According to the method, the instrument, and the program, the complex of the target protein and the compound (e.g., a protein, a low molecular weight compound, and nucleic acid), whose the three-dimensional structure is to be predicted is dissociated, and the fragmentation spectrum is thereby measured. The fragment ion assignment information on both of or one of the target protein and the compound is determined based on the measured fragmentation spectrum. Contact region information in a protein complex on both of or one of the target protein and the compound is determined according to the measured fragmentation spectrum and the determined fragmentation ion assignment information. The three-dimensional structure of both of or one of the target protein and the compound is predicted. Predicted three-dimensional structure prediction data and the determined contact region information are output (e.g., by graphic display or display of a list using a table) while making them correspond to each other. Therefore, upon predicting the three-dimensional structure of a protein (including a gene that encodes the protein) whose three-dimensional structure is unknown, a highly accurate prediction can be performed by adding the actually measured three-dimensional structure information.

A protein structure analysis method, a protein structure analyzing instrument, and a program according to still another aspect of the present invention are characterized in that, at the processing result output step (by the processing result output unit) in the aforementioned protein structure analysis method, protein structure analyzing instrument and program, the three-dimensional structure prediction data is graphically displayed as any one of a wire model, a ribbon model, a pipe model, a ball-and-stick model, and a space filling model for both of or one of the target protein and the compound, and displayed while associating the contact region information with a corresponding displayed region of the three-dimensional structure prediction data.

This shows one example of the processing result output more specifically. According to the method, the instrument, and the program, the three-dimensional structure prediction data is graphically displayed (including, for example, docking simulation) as any one of a wire model, a ribbon model, a pipe model, a ball-and-stick model and a space filling model for both of or one of the target protein and the compound, and further displayed while associating the contact region information with a corresponding displayed region of the three-dimensional structure prediction data (e.g., by link setting, display on the model, display with a specific pattern such as a shading pattern or a slant-line pattern corresponding to the contact region information on the model). Therefore, these pieces of information can be visually and recognizably displayed, and the user can intuitively determine the reliability of the three-dimensional structure prediction data.

A protein structure analysis method, a protein structure analyzing instrument, and a program according to still another aspect of the present invention are characterized in that, at the processing result output step (by the processing result output unit) in the aforementioned protein structure analysis method, protein structure analyzing instrument and program, the three-dimensional structure prediction data of both of or one of the target protein and the compound is displayed in a different color according to its correspondence to the contact region information.

This shows one example of the processing result output more specifically. According to the method, the instrument, and the program, when the three-dimensional structure prediction data is displayed graphically or using a table, the three-dimensional structure prediction data of both of or one of the target protein and the compound is displayed in a different color according to its correspondence to the contact region information. Therefore, these pieces of information can be displayed visually recognizably, and the user can intuitively determine the reliability of the three-dimensional structure prediction data.

A protein structure analysis method, a protein structure analyzing instrument and a program according to still another aspect of the present invention further comprise a predicted structure evaluation information determining step (a predicted structure evaluation information determining unit) of comparing the three-dimensional structure prediction data predicted at the three-dimensional structure predicting step (by the three-dimensional structure predicting unit) with the contact region information determined at the contact region information determining step (by the contact region information determining unit), of evaluating a predicted structure region corresponding to a contact region in a protein complex specified by the contact region information, and of determining predicted structure evaluation information; and a predicted structure evaluation information output step (a predicted structure evaluation information output unit) of outputting the predicted structure evaluation information determined at the predicted structure evaluation information determining step (by the predicted structure evaluation information determining unit).

According to the method, the instrument and the program, the predicted three-dimensional structure prediction data is compared with the determined contact region information; a predicted structure region corresponding to an contact region specified by the contact region information is evaluated; predicted structure evaluation information is determined; and the determined predicted structure evaluation information is output. Therefore, the predicted structure predicted by the calculator can be evaluated based on biochemical experiment data, and prediction accuracy can be remarkably improved.

Further, this feature makes it possible to remarkably improve its accuracy for predicting a function based on the three-dimensional structure in case the functional analysis is to be carried for the interesting gene or protein that is found in a genomic sequence analysis, an expression profiling analysis using a DNA chip, a proteome analysis or the like. In case of the genomic sequence analysis, the function of interest could be more efficiently predicted by performing functional analysis based on the three-dimensional structure of the function-unknown gene or protein. Besides, the model structure and the active site (contact region) information makes it possible to design a drug such as an inhibitor, to improve activity based on protein engineering and to design a functionally modified protein.

A protein structure analysis method, a protein structure analyzing instrument, and a program according to still another aspect of the present invention further comprise a normal vibration-mode analysis step (a normal vibration-mode analysis unit) of calculating a normal vibration for both of or one of the target protein and the compound, wherein at the processing result output step (by the processing result output unit), a calculation result obtained at the normal vibration-mode analysis step (by the normal vibration-mode analysis unit) is displayed in association with the corresponding displayed region of the three-dimensional structure prediction data.

According to the method, the instrument, and the program, the normal vibration is calculated for both of or one of the target protein and the compound, and the normal vibration-mode analysis result (e.g., flexibility information) is displayed in association with the corresponding displayed region of the three-dimensional structure prediction data (e.g., displayed as a vector on the three-dimensional structure model). Therefore, the flexibility information can be considered as the contact region. Namely, by obtaining the flexibility information based on the normal vibration-mode analysis and comparing the flexibility information with the MS measurement data, the prediction model can be evaluated.

A protein structure analysis method, a protein structure analyzing instrument, and a program according to still another aspect of the present invention are characterized in that, in the aforementioned protein structure analysis method, protein structure analyzing instrument and program, at the fragmentation spectrum measuring step (by the fragmentation spectrum measuring unit), the complex of the target protein and the compound is dissociated after a hydrogen-deuterium exchange reaction is provoked for the complex, and the fragmentation spectrum is measured; and at the contact region information determining step (by the contact region information determining unit), a deuteration rate of both of or one of the target protein and the compound is determined according to the fragmentation spectrum measured at the fragmentation spectrum measuring step (by the fragmentation spectrum measuring unit) and the fragment ion assignment information determined at the assignment information determining step (by the assignment information determining unit), a contact region in the protein complex is specified according to the deuteration rate for both of or one of the target protein and the compound, and a contact region on both the target protein and the compound is determined according to the contact region information.

This shows one example of the fragmentation spectrum measurement more specifically. According to the method, the instrument, and the program, the complex of the target protein and the compound is dissociated after a hydrogen-deuterium exchange reaction is provoked for the complex; the fragmentation spectrum is measured; a deuteration rate of both of or one of the target protein and the compound is determined according to the measured fragmentation spectrum and the determined fragment ion assignment information; a contact region of both of or one of the target protein and the compound is specified according to the deuteration rate; and contact region information on both of or one of the target protein and the compound is determined according to the contact region. Therefore, the contact region information can be efficiently obtained.

A protein structure analysis method, a protein structure analyzing instrument, and a program according to still another aspect of the present invention are characterized in that, in the aforementioned protein structure analysis method, protein structure analyzing instrument and program, at the fragmentation spectrum measuring step (by the fragmentation spectrum measuring unit), the complex of the target protein and the compound is dissociated after a chemical modification is provoked for the complex, and the fragmentation spectrum is thereby measured; and at the contact region information in a protein complex determining step (by the contact region information in a protein complex determining unit), a chemically modified region of both of or one of the target protein and the compound is determined according to the fragmentation spectrum measured at the fragmentation spectrum measuring step (by the fragmentation spectrum measuring unit) and the fragment ion assignment information determined at the assignment information determining step (by the assignment information determining unit); a contact region in the protein complex is specified for both of or one of the target protein and the compound according to the chemically modified region, and contact region information on the both of or one of the target protein and the compound is determined according to the contact region.

This shows one example of the fragmentation spectrum measurement more specifically. According to the method, the instrument, and the program, the complex of the target protein and the compound is dissociated after a chemical modification is provoked for the complex, the fragmentation spectrum is thereby measured. Further a chemically modified region of both of or one of the target protein and the compound is determined according to the measured fragmentation spectrum and the determined fragment ion assignment information, an contact region in the protein complex is specified for both of or one of the target protein and the compound according to the chemically modified region, and contact region information on both of or one of the target protein and the compound is determined according to the contact region. Therefore, the solvent accessible information can be efficiently obtained.

A protein structure analysis method, a protein structure analyzing instrument, and a program according to still another aspect of the present invention are characterized in that, at the fragmentation spectrum measuring step (by the fragmentation spectrum measuring unit) in the aforementioned protein structure analysis method, protein structure analyzing instrument and program, the complex of the target protein and the compound is dissociated to the fragment ions by at least one method of collisionally activated dissociation or collision induced dissociation (CID) methods that include a hexapole CID method, a nozzle skimmer CID method, a capillary skimmer CID method, an SORI-CID method, and a multi-pole store assisted capillary skimmer CID method, an IRMPD method, an in-source decay (ISD) method, a post-source decay (PSD) method, a surface-induced dissociation (SID) method, an ECD method, and a BIRD method, and the fragmentation spectrum is measured.

This shows one example of the fragmentation spectrum measurement more specifically. According to the method, the instrument, and the program, the complex of the target protein and the compound is dissociated to the fragment ions by at least one method of collisionally activated dissociation or collision induced dissociation (CID) methods that include a hexapole CID method, a nozzle skimmer CID method, a capillary skimmer CID method, an SORI-CID method and a multi-pole store assisted capillary skimmer CID method, an IRMPD method, an in-source decay (ISD) method, a post-source decay (PSD) method, a surface-induced dissociation (SID) method, an ECD method and a BIRD method, and the fragmentation spectrum is thereby measured. Therefore, the fragment ions that reflect the three-dimensional structure of the target protein can be efficiently generated.

A protein structure analysis method, a protein structure analyzing instrument and a program according to still another aspect of the present invention are characterized in that, at the fragmentation spectrum measuring step (by the fragmentation spectrum measuring unit) in the aforementioned protein structure analysis method, protein structure analyzing instrument and program, the complex of the target protein and the compound is fragmented by proteolysis with enzymatic activity to the fragmented peptides, and the mass spectrum and fragmentation spectrum of the fragmented peptides are, measured.

This shows one example of the fragmentation spectrum measurement more specifically. According to the method, the instrument and the program, by dissociating the complex of the target protein and the compound by an enzymatic reaction, and combining the H/D exchange method or the chemical modification method, the contact region information in a protein complex or the like can be efficiently obtained.

Furthermore, the present invention relates to a recording medium. The recording medium according to the present invention is a recording medium that makes a computer execute the program explained above.

According to this recording medium, by making the computer read and execute the recording medium, the program can be utilized using the computer, and the same advantages as those of the program can be attained.

### BRIEF DESCRIPTION OF THE DRAWINGS

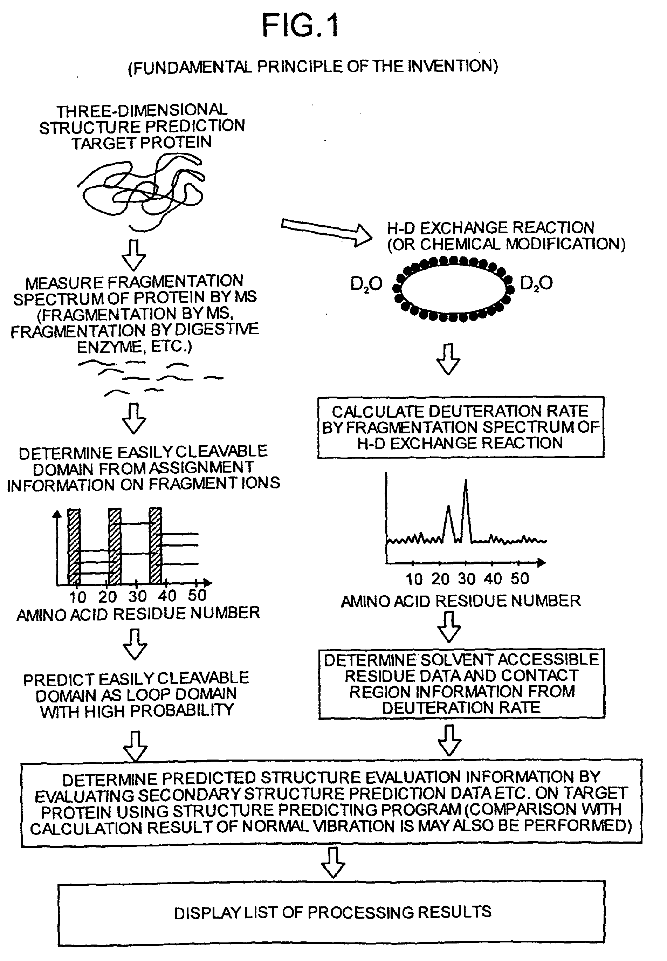

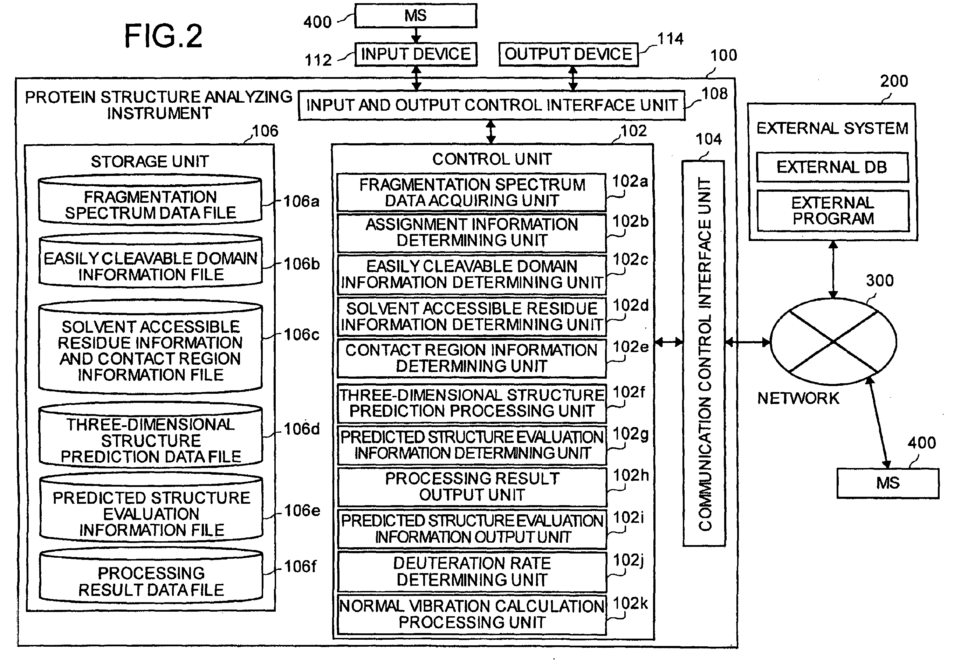

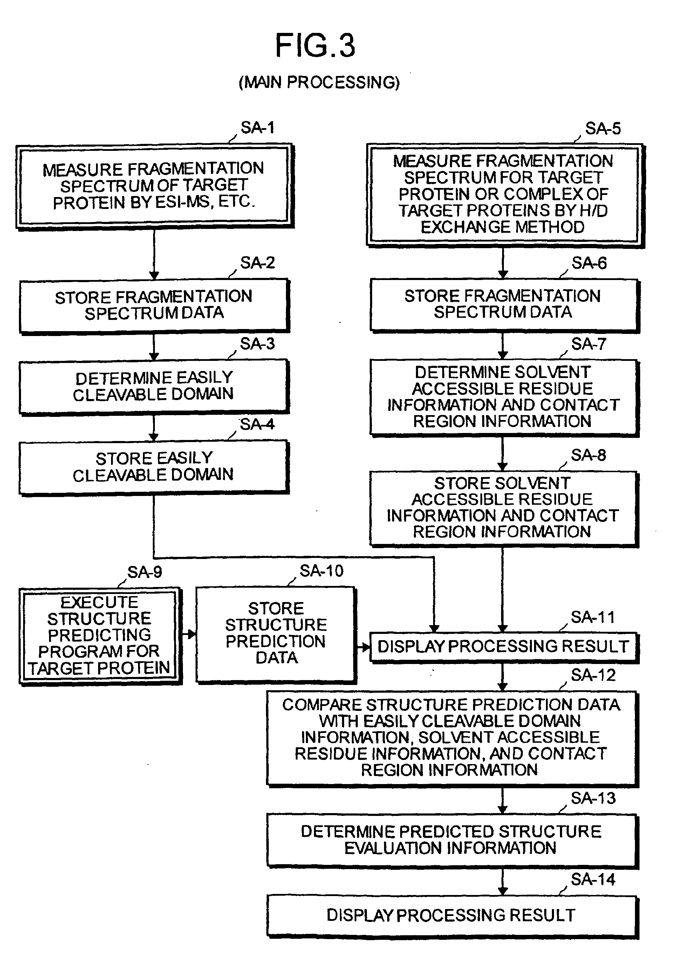

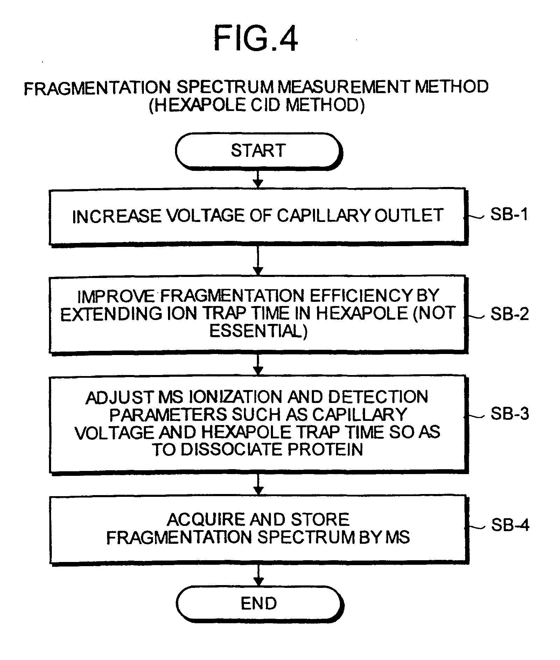

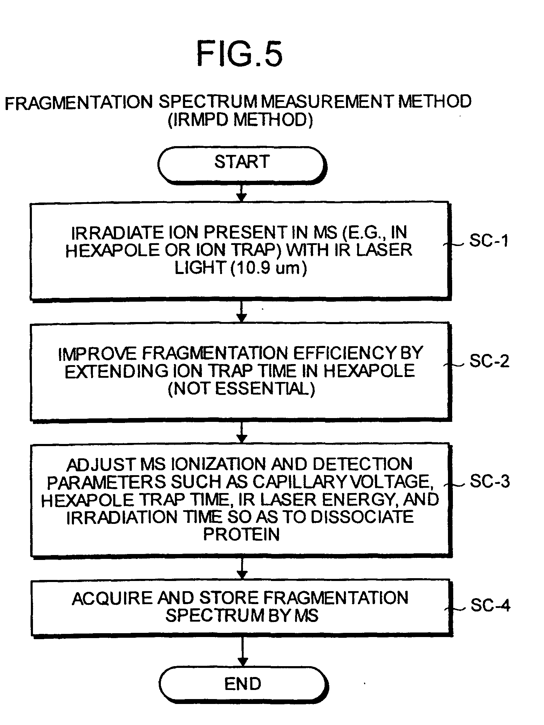

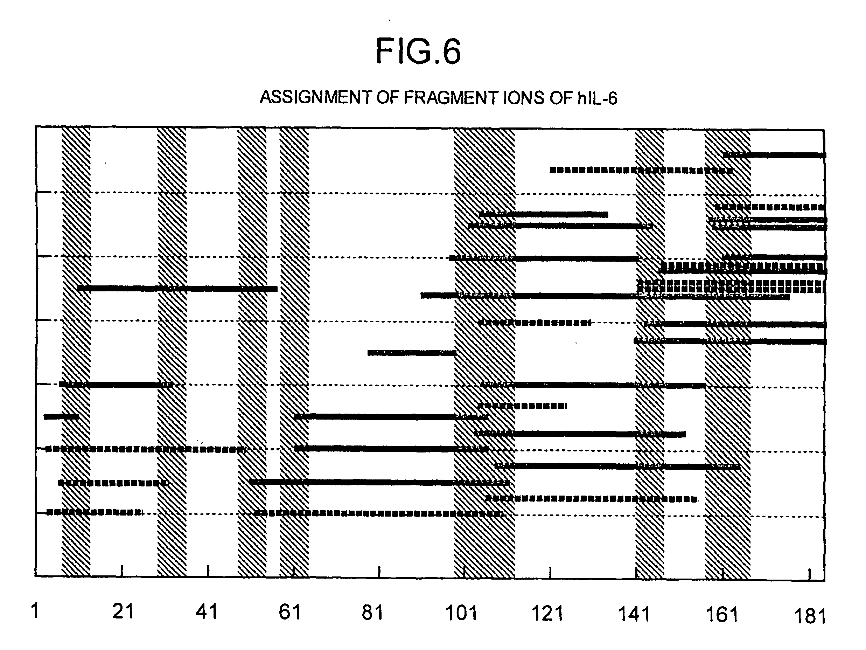

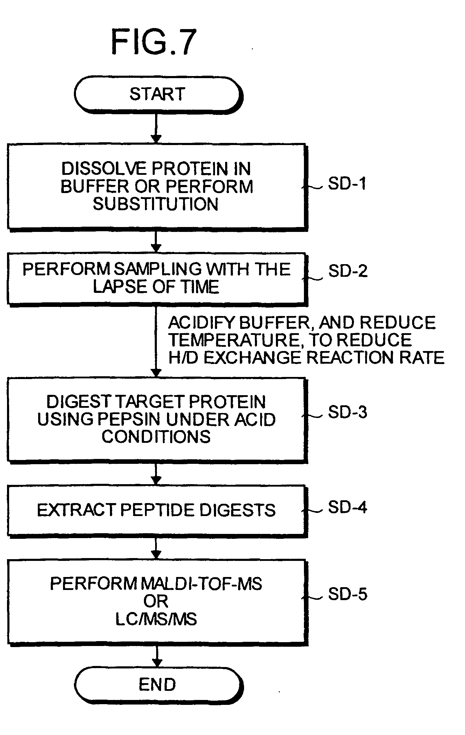

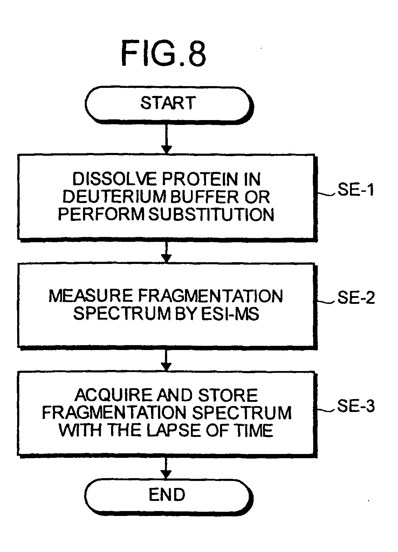

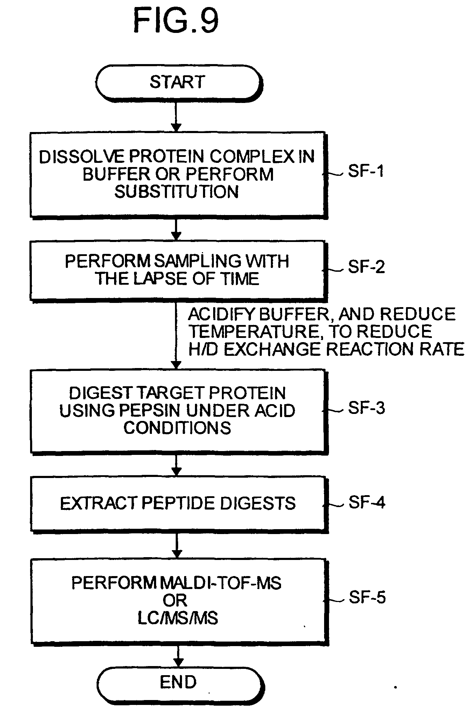

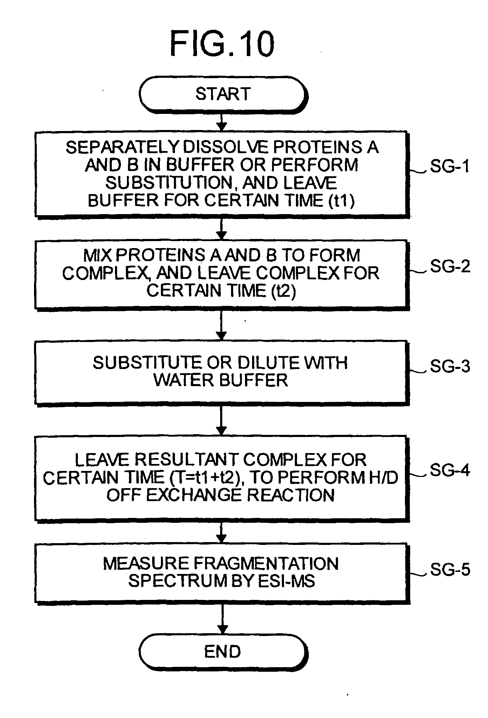

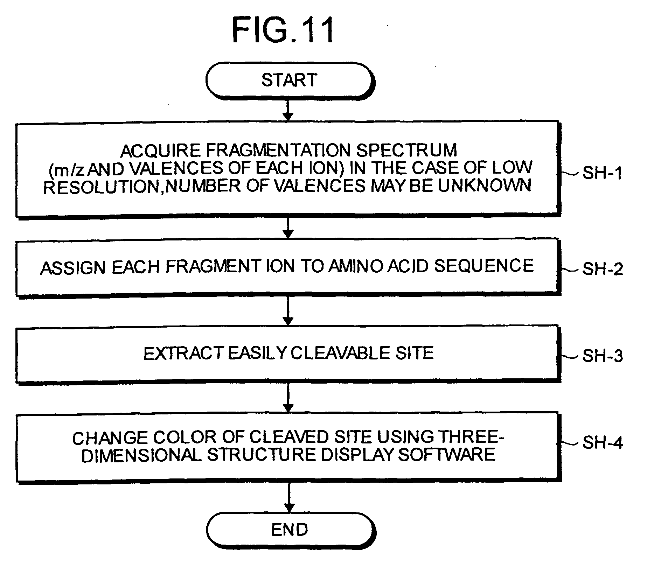

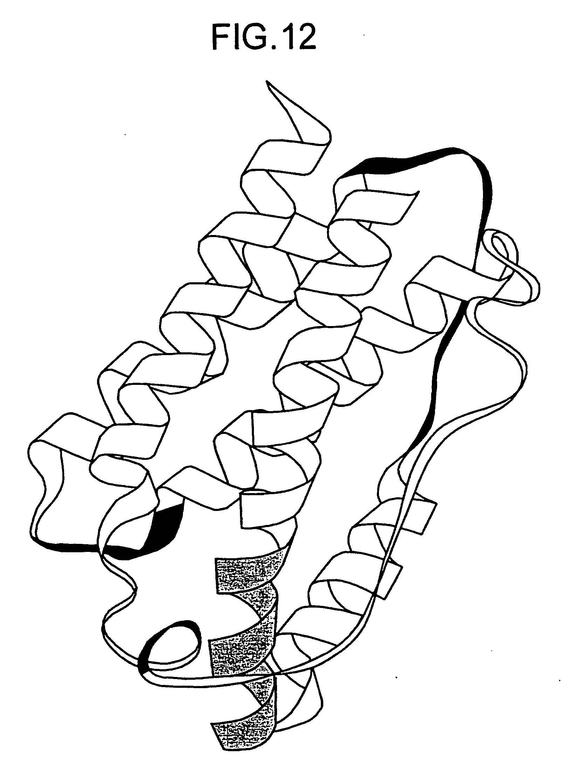

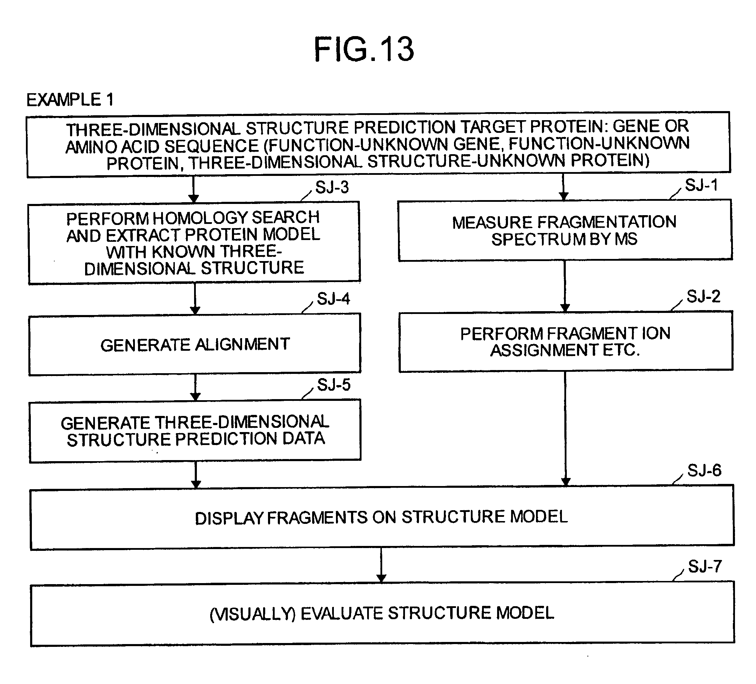

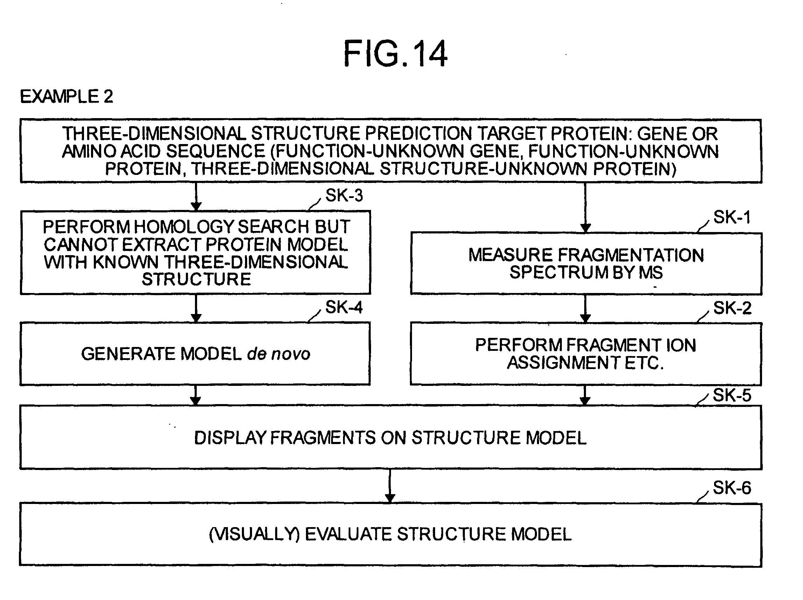

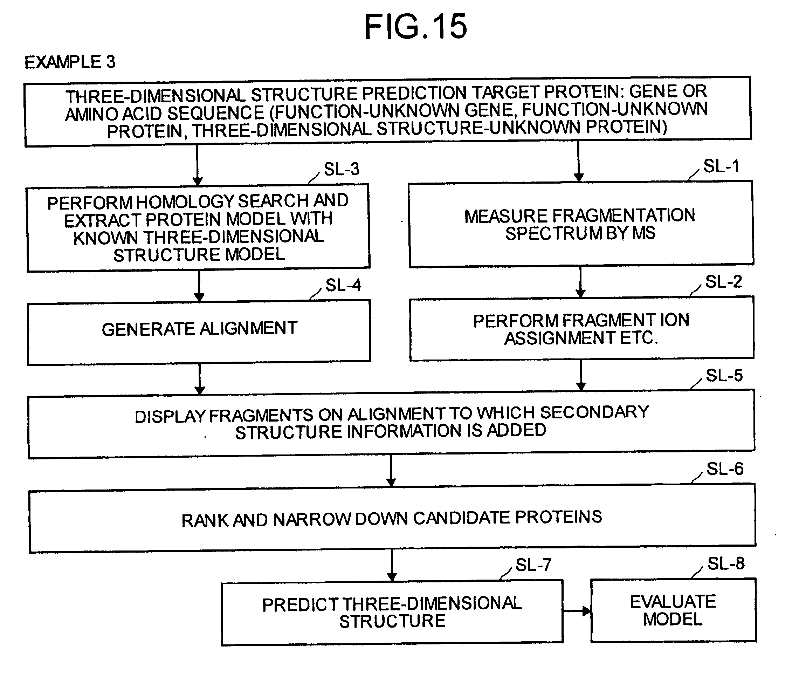

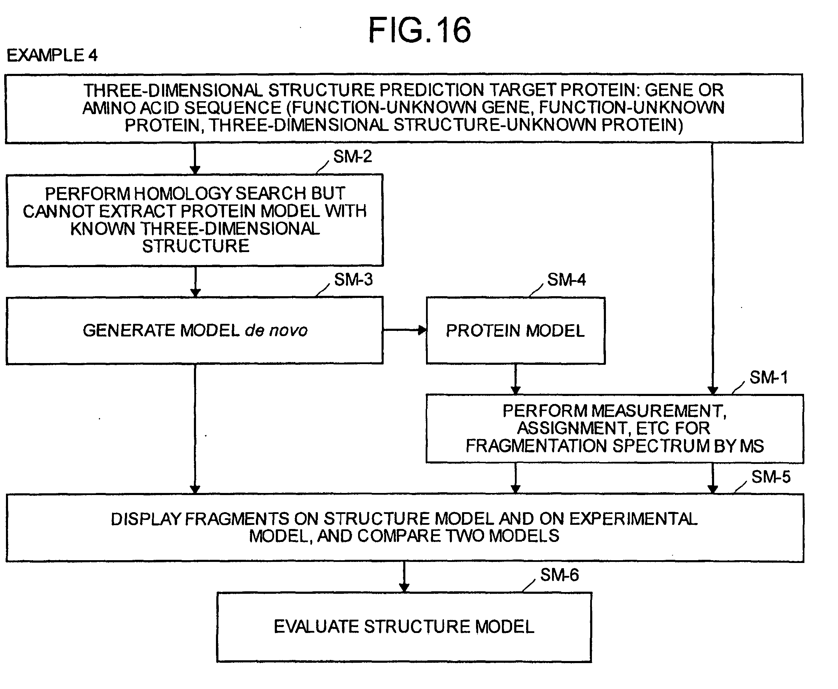

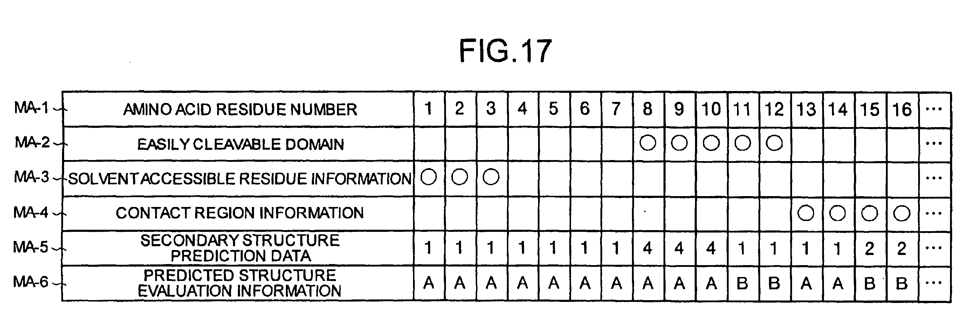

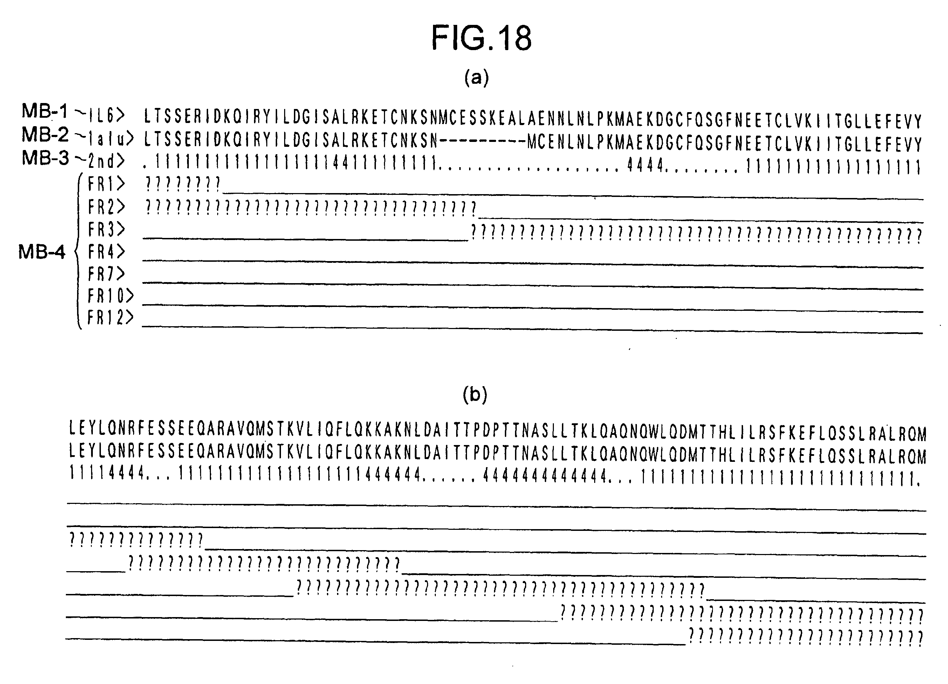

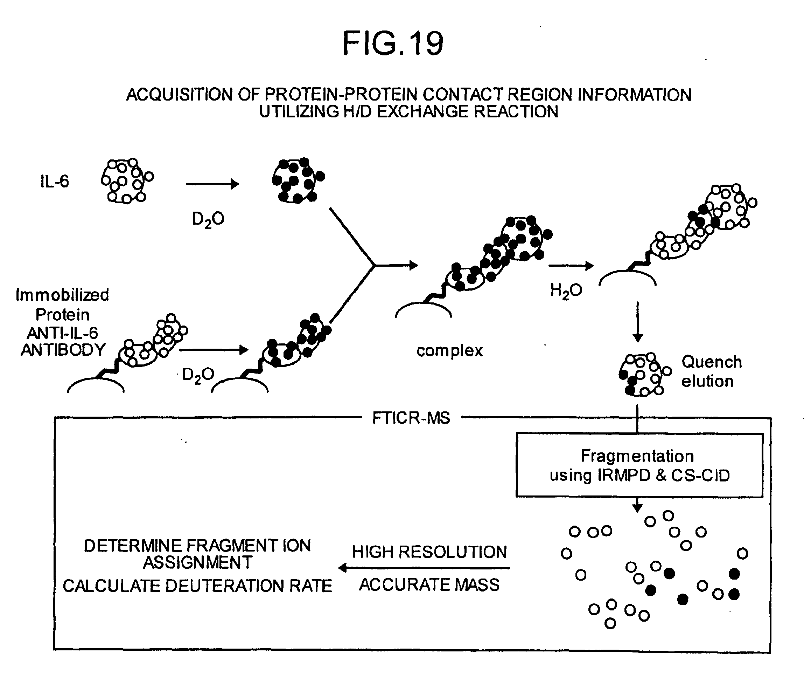

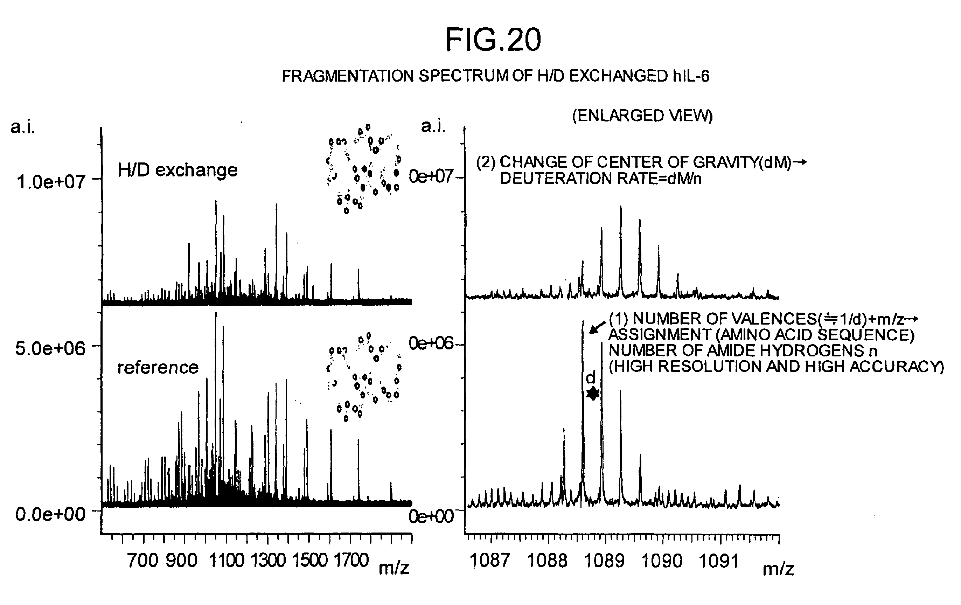

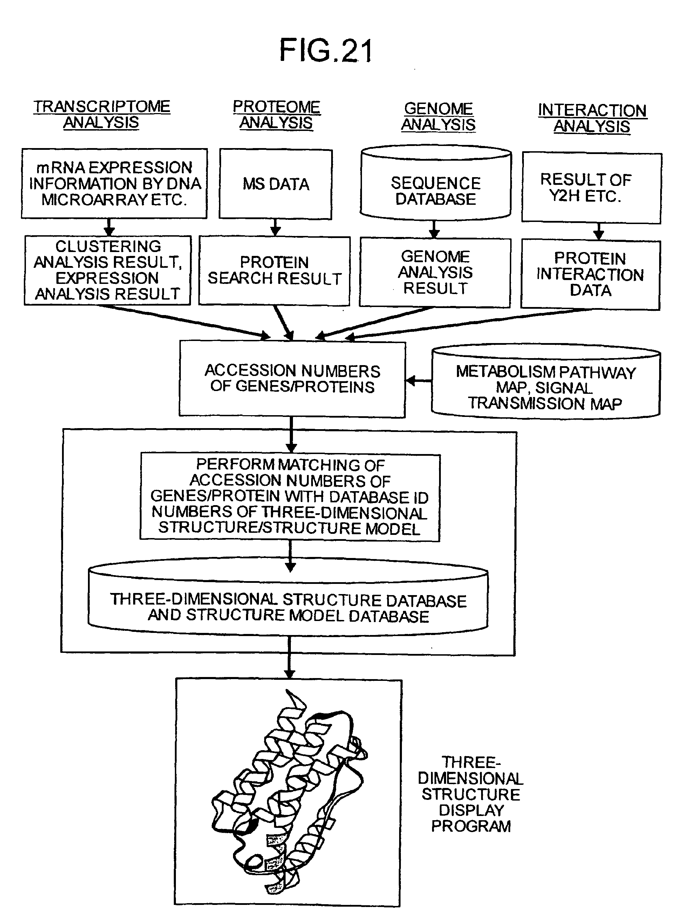

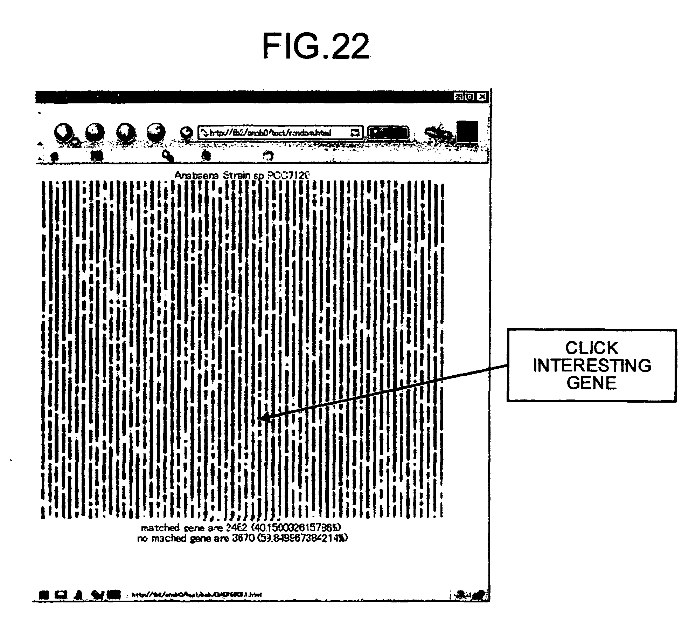

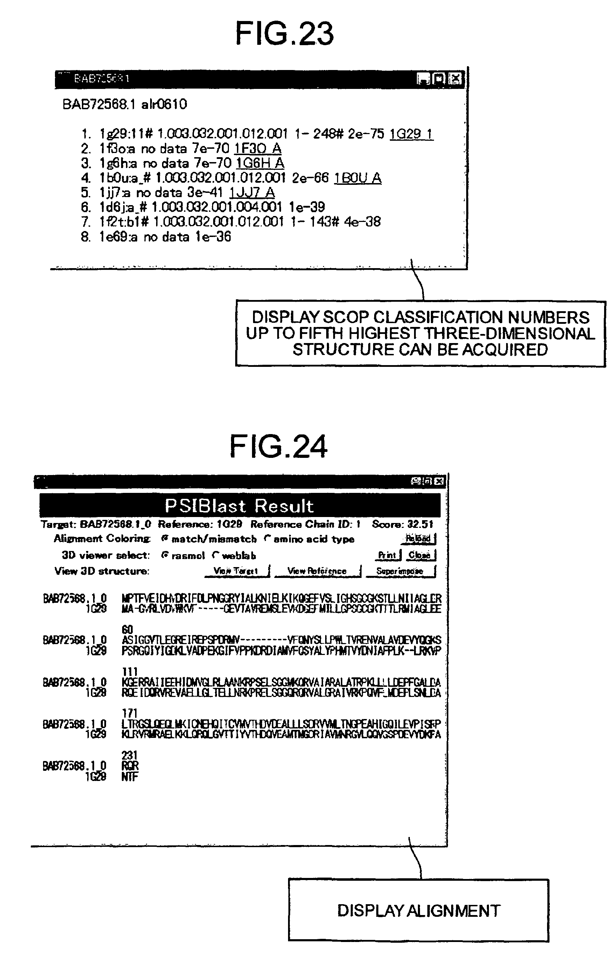

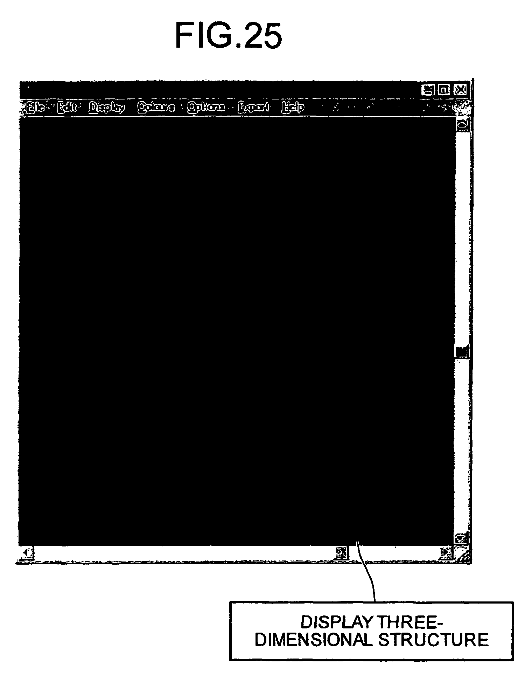

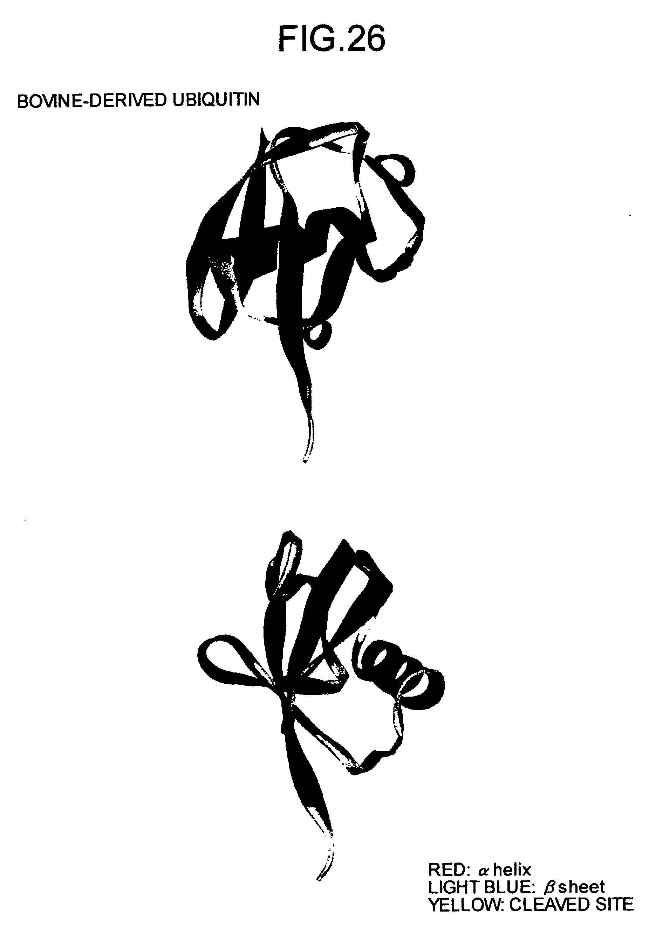

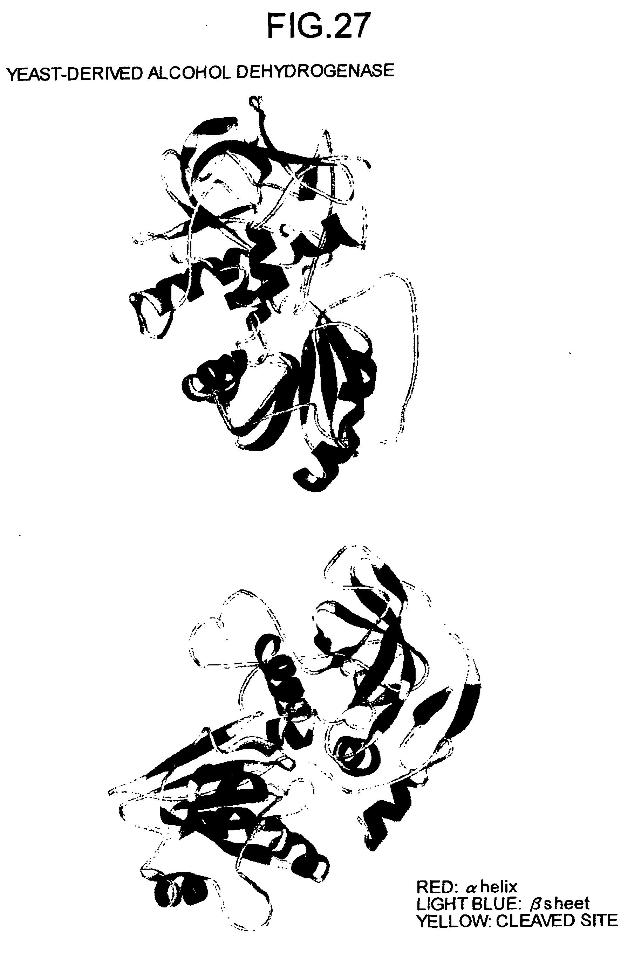

Fig. 1 is a block diagram illustrating the basic principle of the present invention; Fig. 2 is a block diagram illustrating a configuration of a system to which the present invention is applied; Fig. 3 is a flowchart illustrating one example of a main processing of the system according to the present embodiments; Fig. 4 is a flowchart illustrating one example of measurement of a fragmentation spectrum by a hexapole CID method according to the present embodiments; Fig. 5 is a flowchart illustrating one example of measuring the fragmentation spectrum by an IRMPD method according to the present embodiments; Fig. 6 is a conceptual view of obtaining fragment ion assignment information on a human interleukin 6 (IL-6) and determining the easily cleavable domain; Fig. 7 is a flowchart illustrating one example of measuring the fragmentation spectrum by a hydrogen-deuterium exchange method according to the present embodiments; Fig. 8 is a flowchart illustrating one example of merely fragmenting the protein by the MS using the hydrogen-deuterium exchange method and measuring the fragmentation spectrum according to the present embodiments; Fig. 9 is a flowchart illustrating one example of fragmenting a protein complex and measuring the fragmentation spectrum by a hydrogen-deuterium exchange method according to the present embodiments; Fig. 10 is a flowchart illustrating one example of fragmenting a complex of proteins A and B and measuring the fragmentation spectrum by the hydrogen-deuterium exchange method according to the present embodiments; Fig. 11 is a flowchart illustrating one example of analyzing the fragmentation spectrum in Examples; Fig. 12 illustrates one example of a display screen that graphically depicts the three-dimensional structure prediction data for a human interleukin-6 (IL-6) as a ribbon model, and the three-dimensional structure prediction data in different colors according to their correspondence to the easily cleavable domain information; Fig. 13 is a flowchart illustrating one example of the system in Example 1 according to the present invention; Fig. 14 is a flowchart illustrating one example of the system in Example 2 according to the present invention; Fig. 15 is a flowchart illustrating one example of the system in Example 3 according to the present invention; Fig. 16 is a flowchart illustrating one example of the system in Example 4 according to the present invention; Fig. 17 illustrates one example of a display screen that depicts a processing result as a list using a table; Fig. 18 illustrates one example of the processing result in Example 5; Fig. 19 is a conceptual view of provoking a hydrogen-deuterium exchange reaction for a complex of the human interleukin 6 (IL-6) and an anti-IL-6 antibody, fragmenting the complex to fragment ions by an electro-spray ionization method, and measuring the fragmentation spectrum; Fig. 20 is a conceptual view of obtaining a deuteration rate from the fragmentation spectrum of the human IL-6 for which the hydrogen-deuterium reaction is provoked; Fig. 21 is a flowchart illustrating one example of the present system in Example 8 according to the present invention; Fig. 22 illustrates one example of a display screen that depicts the differences in expression amount by the DNA micro-array in different colors; Fig. 23 illustrates a screen of displaying genetic information selected by a user in the present system in Example 8 according to the present invention; Fig. 24 illustrates a display screen of alignments in the present system in Example 8 according to the present invention; Fig. 25 illustrates a display screen of the three-dimensional structure drawn by graphic software in the present system in Example 8 according to the present invention; Fig. 26 illustrates one example of a three-dimensional structure of ubiquitin derived from bovine in the present system in Example 6 according to the present invention; and Fig. 27 illustrates one example of a three-dimensional structure of an alcohol dehydrogenase derived from yeast in the present system in Example 7 according to the present invention.

### BEST MODE FOR CARRYING OUT THE INVENTION

Embodiments of the protein structure analysis method, protein structure analyzing instrument program and recording medium according to the present invention will be explained below in detail with reference to the accompanying drawings. Note that the invention is not limited by the embodiments.

In the following embodiments, an example of applying the present invention to interleukin 6 or the like will be explained. However, the present invention is not limited to this example but can be similarly applied to all proteins.

### Outline of the Invention

An outline of the present invention will be explained hereinafter, and a configuration, processings, and the like of the present invention will subsequently be explained in detail. Fig. 1 is a principle block diagram of a basic principle of the present invention.

In summary, the present invention includes the following basic features. A three-dimensional structure prediction target protein (hereinafter, "target protein") whose three-dimensional structure is to be predicted is dissociated by one of various fragmentation methods using an MS, an enzyme digestion method, and the like, and a fragmentation spectrum is measured.

The target protein is measured using the MS as follows. The target protein is ionized by at least one of spray ionization methods such as an electro-spray ionization method, a laser spray ionization method, a sonic spray ionization method, and an atmospheric pressure chemical ionization method, an MALDI method, and other equivalent ionization methods. Generated ions of the target protein are dissociated to fragment ions by at least one of collisionally activated dissociation or collision induced dissociation (CID) methods including a hexapole CID method (similar to a nozzle skimmer CID method, the capillary skimmer CID method, or a multi-pole store assisted capillary skimmer CID method) and an SORI-CID method, an IRMPD method, an in-source decay (ISD) method, a post-source decay (PSD), a surface-induced dissociation (SID) method, an ECD (an electron capture dissociation) method, a BIRD (a black-body infrared radiative dissociation) method, and other equivalent fragmentation methods. In addition, the fragmentation spectrum is measured. Alternatively, the target protein may be fragmented by the enzymatic digestion method using a pepsin, a trypsin, or the like to the fragmented peptides, and the mass spectrum and fragmentation spectrum of the fragmented peptides, may then be measured.

The capillary skimmer CID method is an in-source fragmentation method that induces ionization and fragmentation using a voltage between a capillary and as skimmer provided in an ion guide (see S. Akashi, Anal. Chem., 71, 4974-4980 (1999)).

The hexapole CID method is a method that improves an efficiency of the capillary skimmer CID method by causing ions to reside in a hexapole for a certain period of time (see K. A., Sannes-Lowery, J. Am. Soc. Mass Spectrom., 11, 1-9 (2000)).

The IRMPD method is a method that induces fragmentation by irradiating ions with a carbon dioxide laser light (see C. P. Dufresne, J. Am. Soc. Mass Spectrom., 9, 1222-1225 (1998)).

For the fragmentation spectrum obtained by using one of these methods, assignment of each fragment is determined. Namely, based on the measured fragmentation spectrum, assignment information on each fragment ion to an amino acid sequence of the target protein is determined. As the amino acid sequence of the target protein, a sequence stored in the PDB may be used if such information is stored therein, or the amino acid sequence of the target protein may be identified using an amino acid sequence identification method by the MALDI-TOF-MS or MS/MS.

Based on the fragment ion assignment information thus determined, a region of the amino acid sequence of the target protein, in which said region of the target protein is dissociated to fragment ions, is specified; and easily cleavable domain information on the amino acid sequence of the target protein is determined according to the specified region.

Fig. 6 is a conceptual view of obtaining fragment ion assignment information on a human interleukin 6 (IL-6) and determining the easily cleavable domain information. In Fig. 6, a fragment of the fragment ion obtained when fragmentation is performed by the IRMPD method is represented by a dotted line, and that obtained when fragmentation is performed by the hexapole CID method is represented by a solid line. In addition, easily cleavable domain information obtained based on a cleaved sites of these fragment ions is denoted by a slant line.

A three-dimensional structure of the target protein is then predicted. A three-dimensional structure prediction may be performed by any one of existing three-dimensional structure predicting methods such as a homology modeling method, a molecular simulation method, an ab initio method, a secondary structure predicting method, a 3D-1D method, and a threading method. One example of a three-dimensional structure prediction scheme based on sequence homology will be explained.

A search of an amino acid sequence of the target protein (as to gene, an amino acid sequence obtained from the DNA sequence) whose three-dimensional structure is to be predicted, and a similar amino acid sequence whose three-dimensional structure is known may be conducted using a computer program such as PASTA (Pearson WR, Methods Enzymol, 266, 227-258, 1996), PSI-BLAST (SchafferAA, Wolf YI, Ponting CP, Koonin EV, Aravind L and Altschul SF, Bioinformatics, 12, 1000-1011, 1999), LIBRA (Ota, M. and Nishikawa, K., Protein Engineering, 10, 339-351, 1997), RBS-BLAST (Schaffer AA, Wof YI, Ponting CP, Koonin EV, Aravind L and Altschul SF, Bioinformatics, 12, 1000-1011, 1999), IMPALA (A. A. Schaffer et al., Bioinformatics, 15, (12), 1000-1011, 1999), HMMER (R. Durbin, S. Eddy, A. Krogh, and G. Mitchison, Cambridge University Press, 1998, S. R. Eddy. Bioinformatics, 14, 755-763, 1998), or ClustalW (Thompson, J. D., D. G. Higgins, and T. J. Gibson, Nucleic Acids Res. 22, 4673-4680, 1994).

As the search target, a database obtained by processing the PDB in advance is preferably used. The processed database is created by classifying all of protein sequences registered in the PDB to classes having 95% or more of the sequence homology, and by extracting the protein sequence with a highest experimental accuracy in each class as a typical sequence. Use of this processed database enables increase of an efficiency of the homology search, and ensuring diversity and variety of three-dimensional structures to be searched even if the number of searched items is restricted.

Based on the output result by the search program, pairwise alignments between the target protein and the reference protein sequences are created individually by as much as the number of searched items. A secondary structure domain in the three-dimensional structure of each reference protein is identified for each pairwise alignment, and reference is made therefor onto the reference protein sequence. Alternatively, multiple alignments may be performed based on the target protein and a plurality of reference protein sequences.

Further, reference is made onto an alignment result on which secondary structure information is displayed, for the easily cleavable domain information obtained from the fragmentation data by MS. Namely, both ends of the fragment ion obtained by MS are easily cleavable sites and therefore highly possibly correspond to high motility loops on the three-dimensional structure. A region between the both ends of the fragment ion obtained by MS is a site that is not easily dissociated and, therefore, highly possibly corresponds to a site that includes a secondary structure such as the α helix or the β strand.

Candidate proteins are evaluated (given ranks) taking all pieces of fragment ion information into consideration.

The three-dimensional structure prediction is then performed as to the target protein by the homology modeling method based on the three-dimensional structure of the highly evaluated candidate protein. As a three-dimensional structure prediction program, a "FAMS" (Ogata, K. and Umeyama, H., J. Mol. Graphics Mod. 18, 258-272, 2000), Swiss-Model (Peitsch MC., Biochem. Soc. Trans. 24, 274-279, 1996), CPHmodels (O. Lund, K. Frimand, J. Gorodkin, H. Bohr, J. Bohr, J. Hansen, and S. Brunak., Protein Engineering, 10, 1241-1248, 1997), "SAM-T98" (J. Park, K. Karplus, C. Barrett, R. Hughey, D. Haussler, T. Hubbard, and C. Chothia, JMB 284 (4), 1201-1210, 1998), "MODELLER" (A. Sali, L. Potterton, F. Yuan, H. van Vlijmen, and M. Karplus, Proteins, 23, 318-326, 1995) or the like may be used. Furthermore, other three-dimensional structure prediction programs such as one disclosed or referred in "CAFASP2 The Second Critical Assessment of Fully Automated Structure Prediction Methods, PROTEINS, 5;171-183 (2001)" by Daniel Fischer et al., may also be used.

For each candidate protein, the predicted three-dimensional structure prediction data and the determined easily cleavable domain information are output (e.g., graphically displayed, displayed as a list using a table) while making them correspond to each other. For example, the three-dimensional structure prediction data may be graphically displayed as any one of a wire model, a ribbon model, a pipe model, a ball-and-stick model, and a space filling model. In addition, the three-dimensional structure prediction data may be displayed while associating the easily cleavable domain information with a corresponding displayed region of the three-dimensional structure prediction data (e.g., by link setting, display on the model, display with a specific pattern such as a shading pattern or a slant-line pattern corresponding to the easily cleavable domain information on the model). Further, in the graphic display or table display, the three-dimensional structure prediction data may be displayed in a different color according to its correspondence to the easily cleavable domain information.

The predicted three-dimensional structure prediction data is compared with the determined easily cleavable domain information. The predicted structure region corresponding to the easily cleavable domain specified by the easily cleavable domain information is evaluated, and predicted structure evaluation information is determined. The determined predicted structure evaluation information is then output. That is, reference is made for the obtained predicted structure and the MS fragmentation data, thereby the predicted structure is evaluated.

Further, for example, for the high homology candidate protein, the fragmentation spectrum is measured by MS and the measured fragmentation spectrum is compared with that of the target protein, whereby the predicted structure can be evaluated more accurately.

Furthermore, according to the present invention, a hydrogen-deuterium exchange reaction is provoked for the target protein or complex, thereby fragmenting the protein or complex to fragment ions and measuring the fragmentation spectrum.

Fig. 19 is a conceptual view of provoking a hydrogen-deuterium exchange reaction for a complex of the human interleukin 6 (IL-6) and an anti-IL-6 antibody, fragmenting the complex to fragment ions by the electro-spray ionization method, and measuring the fragmentation spectrum.

Based on the measured fragmentation spectrum, fragment ion assignment information on the amino acid sequence of the target protein is determined, and a deuteration rate of each amino acid residue in the amino acid sequence of the target protein is determined according to the measured fragmentation spectrum and the determined fragment ion assignment information.

Fig. 20 is a conceptual view of obtaining the deuteration rate from the fragmentation spectrum of the human IL-6 for which the hydrogen-deuterium reaction is provoked. As shown in Fig. 20, the deuteration ratio (D ratio) is determined according to changes in centers of gravity of the fragmentation spectrum of the human IL-6 for which the hydrogen-deuterium reaction is provoked (upper views) and of the fragmentation spectrum of the human IL-6 for which the hydrogen-deuterium reaction is not provoked (lower views). The deuteration rate is calculated from deuteration ratio obtained with the lapse of time.

In accordance with the determined deuteration rate of each amino acid residue in the amino acid sequence, a region of the amino acid sequence of the target protein which region is in contact with a solvent is specified, and solvent accessible residue information on the amino acid sequence of the target protein is determined according to the specified region. For example, a site having a slower deuteration rate than that measured in a control experiment in which no hydrogen-deuterium exchange reaction is provoked may be determined as the solvent accessible residue information.

For the complex, contact region information in a protein complex on the amino acid sequence of the target protein is determined according to the determined deuteration rate of each amino acid residue in the amino acid sequence. For example, a site having a slower deuteration rate than that measured when the complex is not formed may be determined as the contact region information in a protein complex. Further, a site having a slower deuteration rate than that measured in a control experiment in which no hydrogen-deuterium exchange reaction is provoked may be determined as the contact region information in a protein complex.

The solvent accessible residue information and contact region information thus determined are output together with the prediction data or used in determination of the predicted structure evaluation information. In other words, the predicted three-dimensional structure prediction data, the determined solvent accessible residue information, and the determined contact region information are output while making them correspond to one another (e.g., by graphic display or display of a list using a table). Further, the predicted three-dimensional structure prediction data is compared with the solvent accessible residue information and the contact region information thus determined, and the predicted structure region that corresponds to a solvent accessible residue specified by the solvent accessible residue information or an interactive interface specified by the contact region information is evaluated. The predicted structure evaluation information is thereby determined and output.

Fig. 12 illustrates one example of a display screen that graphically displays the three-dimensional structure prediction data for the human interleukin-6 (IL-6) as a ribbon model, and the three-dimensional structure prediction data are displayed in different colors according to its correspondence to the easily cleavable domain information. In Fig. 12, the easily cleavable domain information is indicated in black, and the contact region information is indicated in gray.

Fig. 17 illustrates one example of a display screen that displays a processing result as a list using a table. As shown in this figure, a processing result list display screen includes, for example, a display region MA-1 for displaying an amino acid residue number of the protein, a display region MA-2 for displaying the easily cleavable domain determined according to the present invention, a display region MA-3 for displaying the solvent accessible residue information determined according to the present invention, a display region MA-4 for displaying the contact region information determined according to the present invention, a display region MA-5 for displaying the three-dimensional structure prediction data (e.g., the secondary structure) by the structure prediction scheme, and a display region MA-6 for displaying the predicted structure evaluation information on the three-dimensional structure prediction data determined according to the present invention. As shown in this figure, these pieces of information may be displayed as a list in a table form for each amino acid residue of the protein. If the contact region information is displayed, it is preferable to display the information while discriminating one-to-many interactions (e.g., a manner of display such as A, B, C, ... may be employed).

Factors for the easily cleavable domain or an easily H/D exchangeable domain are considered to include flexibility information. According to the present invention, therefore, a normal vibration is calculated as the flexibility information, and a normal vibration-mode analysis result is compared with MS data, whereby a model evaluation or the like can be performed. Thus, according to the present invention, the normal vibration calculation is performed for the target protein using one of various known computer programs (e.g., a molecular orbital calculation program 'MOPAC' (product name) manufactured by Fujitsu Limited⁽™⁾), and the normal vibration-mode analysis result (e.g., the flexibility information) is displayed while associating the result with the corresponding displayed region of the three-dimensional structure prediction data (e.g., displayed as a vector on the three-dimensional structure model). It is, therefore, possible to consider the flexibility information in determining the easily cleavable domain. That is, by obtaining the flexibility information from the normal vibration-mode analysis, and comparing the flexibility information with the MS measurement data, the predicted model can be evaluated.

### SYSTEM CONFIGURATION

A configuration of a system according to the present invention will be explained. Fig. 2 is a block diagram of the configuration of the system to which the present invention is applied. In this figure, only parts relating to the present invention are conceptually shown in the system configuration. Schematically, the system is constituted of a protein structure analyzing instrument 100 and an external system 200 that provides an external database on sequence information or the like, and an external program for a homology search or the like, and a network 300 through which the instrument 100 and the system 200 are communicably connected. The protein structure analyzing instrument 100 is constituted so as to be able to acquire fragmentation spectrum information from a mass spectrometer 400 through an input device 112, the network 300, or the like.

In Fig. 2, the network 300 functions to mutually connect the protein structure analyzing instrument 100 to the external system 200 and is, for example, the Internet.

In Fig. 2, the external system 200 is connected to the protein structure analyzing instrument 100 through the network 300, and the external system 200 functions to provide a user with the external database on the sequence information or the like and a website for executing the external program for a homology search, a motif search, or the like.

The external system 200 may be constituted as a WEB server, an ASP server, or the like, and a hardware configuration of the external system 200 may be constituted by an information processing instrument such as a commercially available workstation or personal computer and peripheries thereof. Respective functions of the external system 200 are realized by a CPU, a disk device, a memory device, an input device, an output device, a communication controller, and the like included in the hardware configuration of the external system 200 and a program or the like that controls these devices.

In Fig. 2, the mass spectrometer 400 perform fragmentation of a protein to fragment ions and measures a fragmentation spectrum by one of the methods such as the collisionally activated dissociation or collision induced dissociation (CID) methods including the hexapole CID method, the nozzle skimmer CID method, the capillary skimmer CID method, the SORI-CID method, and a multi-pole store assisted capillary skimmer CID method the IRMPD method, the in-source decay (ISD) method, the post-source decay (PSD), the surface-induced dissociation (SID) method, the ECD method, or the BIRD method.

In Fig. 2, the protein structure analyzing instrument 100 schematically includes a control unit 102, e.g., a CPU, that collectively controls an entirety of the protein structure analyzing instrument 100, a communication interface unit 104 connected to a communication device (not shown), e.g., a router, connected to a communication line or the like, an input and output control interface unit 108 that is connected to the input device 112 and the output device 114, and a storage unit 106 that stores various databases and tables. The respective units are communicably connected to one another through arbitrary communication paths. This protein structure analyzing instrument 100 is communicably connected to the network 300 through the communication device such as the router and a wired or wireless communication line such as a dedicated line.

The various databases, files, and tables (a fragmentation spectrum data file 106a to a processing result data file 106f) stored in the storage unit 106 are storage units such as fixed disk devices. They store various programs, tables, files, databases, webpage files, and the like used for various processings.

Among the constituent elements of the storage unit 106, the fragmentation spectrum data file 106a serves as a fragmentation spectrum data storage unit that stores fragmentation spectrum data acquired from the mass spectrometer 400.

An easily cleavable domain information file 106b serves as an easily cleavable domain information storage unit that stores easily cleavable domain information and the like.

A solvent accessible residue information and contact region information file 106c serves as an contact region information storage unit that stores solvent accessible residue information, contact region information, and the like.

A three-dimensional structure prediction data file 106d serves as a three-dimensional structure prediction data storage unit that stores three-dimensional structure prediction data predicted by a three-dimensional structure prediction processing.

A predicted structure evaluation information file 106e serves as a predicted structure evaluation information storage unit that stores predicted structure evaluation information and the like.

The processing result data file 106f serves as a processing result data storage unit that stores information on processing results and the like.

The storage unit 106 of the protein structure analyzing instrument 100 may record, as other information, a protein information database that stores amino acid sequence information and structure information on proteins or the like. This protein information database or the like may be an external database, e.g., the PDB, accessible through the Internet or an in-house database created by copying these databases, storing original sequence information and the like, or by adding individual clustering information, annotation information, or the like.

In Fig. 2, the communication control interface unit 104 controls a communication between the protein structure analyzing instruments 100 and the network 300 (or the communication device such as the router). Namely, the communication control interface unit 104 functions to communicate data with the other terminal through a communication line.

In Fig. 2, the input and output control interface unit 108 controls the input device 112 and the output device 114. The output device 114 may include a monitor (including a home-use television set), and may also include a loudspeaker (note that the output device 114 is sometimes referred to as the monitor). As the input device 112, a keyboard, a mouse, a microphone or the like can be used. The monitor in cooperation with the mouse realizes a pointing device function.

In Fig. 2, the control unit 102 includes an internal memory that stores a control program such as an operating system (OS), a program that specifies various processing procedures or the like, and memory for storing necessary data. The control unit 102 performs information processings for executing various processings using these programs and the like. In terms of functional concept, the control unit 102 includes a fragmentation spectrum data acquiring unit 102a, an assignment information determining unit 102b, an easily cleavable domain information determining unit 102c, a solvent accessible residue information determining unit 102d, an contact region information determining unit 102e, a three-dimensional structure prediction processing unit 102f, a predicted structure evaluation information determining unit 102g, a processing result output unit 102h, a predicted structure evaluation information output unit 102i, a deuteration rate determining unit 102j, and a normal vibration-mode analysis processing unit 102k.

Among these constituent elements of the control unit 102, the fragmentation spectrum data acquiring unit 102a serves as a fragmentation spectrum data acquiring unit that acquires the fragmentation spectrum data from the mass spectrometer 400 that performs fragmentation of the target protein using the MS, an enzyme, or the like, and measures the fragmentation spectrum.

The assignment information determining unit 102b serves as an assignment information determining unit that determines fragment ion assignment information on the amino acid sequence of the target protein based on the fragmentation spectrum data acquired by the fragmentation data acquiring unit.

The easily cleavable domain information determining unit 102c serves as an easily cleavable domain information determining unit that specifies a region of the amino acid sequence of the target protein, in which said region of the target protein is dissociated to fragment ions, based on the fragment ion assignment information determined at the assignment information determining step, and then determines easily cleavable domain information on the amino acid sequence of the target protein according to the specified region.

The solvent accessible residue information determining unit 102d serves as a solvent accessible residue information determining unit that specifies a region of the amino acid sequence of the target protein which region is in contact with a solvent according to the deuteration rate of each amino acid residue in the amino acid sequence which rate is determined by the deuteration rate determining unit, and then determines solvent accessible residue information on the amino acid sequence of the target protein according to the specified region.

The contact region information determining unit 102e serves as an contact region information determining unit that determines contact region information on the amino acid sequence of the target protein according to the deuteration rate of each amino acid residue in the amino acid sequence which rate is determined by the deuteration rate determining unit.

The three-dimensional structure prediction processing unit 102f serves as a three-dimensional structure prediction processing unit that predicts three-dimensional structures of the target protein and candidate proteins using the existing three-dimensional structure predicting method such as the homology modeling method, the molecular simulation method, the ab initio method, the secondary structure predicting method, the 3D-1D method, or the threading method.

The predicted structure evaluation information determining unit 102g may serve as a predicted structure evaluation information determining unit that compares the three-dimensional structure prediction data predicted by the three-dimensional structure predicting unit with the easily cleavable domain information determined by the easily cleavable domain information determining unit, that evaluates a predicted structure region corresponding to the easily cleavable domain specified by the easily cleavable domain information, and that determines predicted structure evaluation information. The predicted structure evaluation information determining unit 102g may also serve as a predicted structure evaluation information determining unit that compare the three-dimensional structure prediction data predicted by the three-dimensional structure predicting unit with the solvent accessible residue information determined by the solvent accessible residue information determining unit, that evaluates a predicted structure region corresponding to the solvent accessible residue specified by the solvent accessible residue information, and that determines predicted structure evaluation information. Further, the predicted structure evaluation information determining unit 102g may also serve as a predicted structure evaluation information determining unit that compare the three-dimensional structure prediction data predicted by the three-dimensional structure predicting unit with the contact region information determined by the contact region information determining unit, that evaluates a predicted structure region corresponding to the interactive interface specified by the contact region information, and that determines predicted structure evaluation information.

The processing result output unit 102h may serve as a processing result output unit that outputs the three-dimensional structure prediction data predicted by the three-dimensional structure predicting unit and the easily cleavable domain information determined by the easily cleavable domain information determining unit while making them correspond to each other. The processing result output unit 102h may also serve as a processing result output unit that outputs the three-dimensional structure prediction data predicted by the three-dimensional structure predicting unit and the solvent accessible residue information determined by the solvent accessible residue information determining unit while making them correspond to each other. Further, the processing result output unit 102h may also serve as a processing result output unit that outputs the three-dimensional structure prediction data predicted by the three-dimensional structure predicting unit and the contact region information determined by the contact region information determining unit while making them correspond to each other.

The predicted structure evaluation information output unit 102i serves as a predicted structure evaluation information output unit that outputs the predicted structure evaluation information determined by the predicted structure evaluation information determining unit.

The deuteration rate determining unit 102j serves as a deuteration rate determining unit that determines the deuteration rate of each amino acid residue in the amino acid sequence of the target protein according to the fragmentation spectrum data measured by the fragmentation spectrum data acquiring unit and the fragment ion assignment information determined by the assignment information determining unit.

The normal vibration-mode analysis processing unit 102k serves as a normal vibration-mode analysis processing unit that calculates a normal vibration for the target protein. Details of processings performed by these units will be explained later.

### SYSTEM PROCESSINGS

One example of processings of the system according to the present embodiment constituted as explained above will be explained in detail mainly with reference to Fig. 3.

### MAIN PROCESSING

A main processing will first be explained in detail with reference to Fig. 3. Fig. 3 is a flowchart of one example of the main processing of the system according to the present embodiment.

The mass spectrometer 400 measures the fragmentation spectrum of the target protein (at a step SA-1). Namely, a target protein solution is continuously injected into the mass spectrometer 400 using a syringe pump, the target protein is dissociated to fragment ions by the hexapole CID method or the like, and the fragmentation spectrum is measured.

Fig. 4 is a flowchart of one example of measurement of the fragmentation spectrum by the hexapole CID method according to the present embodiment.

A voltage of a capillary outlet is increased (at a step SB-1).

An ion trap time in the hexapole may be extended so as to improve a fragmentation efficiency (at a step SB-2).

Parameters of MS ionization and detection (e.g., the capillary voltage and the trap time in the hexapole) are adjusted so as to perform fragmentation of the protein (at a step SB-3).

The fragmentation spectrum is acquired and stored by the MS (at a step SB-4).

Fig. 5 is a flowchart of an example of measuring the fragmentation spectrum by the IRMPD method according to the present embodiment.

The ions present in the MS (e.g., in the hexapole or ion trap) are irradiated with a laser light (10.9 micrometers (at a step SC-1).

An ion trap time in the hexapole may be extended so as to improve the fragmentation efficiency (at a step SC-2).

Parameters of MS ionization and detection (e.g., the capillary voltage, the trap time in the hexapole, an IR laser energy, and an IR laser irradiation time) are adjusted so as to perform fragmentation of the protein (at a step SC-3).

The fragmentation spectrum is acquired and stored by the MS (at a step SC-4).

The fragmentation spectrum may be acquired by other methods such as the nozzle skimmer CID method, the capillary skimmer CID method, the multi-pole store assisted capillary skimmer CID method, the ECD method, the BIRD method, or the SORI-CID method.

Referring back to Fig. 3, the protein structure analyzing instrument 100 acquires the fragmentation spectrum data from the mass spectrometer 400 through the input device 112 or the network 300 by the processing performed by the fragmentation spectrum data acquiring unit 102a, and stores the fragmentation spectrum data in the fragmentation spectrum data file 106a (at a step SA-2).

The protein structure analyzing instrument 100 determines the fragment ion assignment information on the amino acid sequence of the target protein based on the measured fragmentation spectrum by the processing performed by the assignment information determining unit 102b. In addition, the protein structure analyzing instrument 100 specifies a region of the amino acid sequence of the target protein, in which said region of the target protein is dissociated to fragment ions, based on the determined fragment ion assignment information by the processing performed by the easily cleavable domain information determining unit 102c, and determines the easily cleavable domain information on the amino acid sequence of the target protein according to the specified region (at a step SA-3).

The protein structure analyzing instrument 100 stores the easily cleavable domain information in the easily cleavable domain information file 106b by the processing performed by the easily cleavable domain information determining unit 102c (at a step SA-4).

The protein structure analyzing instrument 100 also measures the fragmentation spectrum for the target protein or the complex thereof by the hydrogen-deuterium exchange method (at a step SA-5).

Fig. 7 is a flowchart of an example of measuring the fragmentation spectrum by the hydrogen-deuterium exchange method according to the present embodiment.

The target protein is dissolved in a buffer or subjected to deuterium substitution (at a step SD-1).

While performing sampling with the lapse of time, the buffer is acidified and the temperature is reduced, to reduce an H/D exchange reaction rate (at a step SD-2).

The target protein is digested using a pepsin under acid conditions (at a step SD-3).

Peptide digests are extracted (at a step SD-4), and the MALDI-TOF-MS or LC/MS/MS is performed (at a step SD-5).

Fig. 8 is a flowchart of an example of fragmenting the protein merely by MS using the hydrogen-deuterium exchange method and measuring the fragmentation spectrum.

The target protein is dissolved in the buffer or subjected to deuterium substitution (at a step SE-1).

The fragmentation spectrum is measured by the ESI-MS (at a step SE-2).

While performing sampling with the lapse of time, the fragmentation spectrum is acquired and stored (at a step SE-3).

Fig. 9 is a flowchart of an example of dissociating a protein complex and measuring the fragmentation spectrum by the hydrogen-deuterium exchange method according to the present embodiment.

The protein complex is dissolved in the buffer or subjected to deuterium substitution (at a step SF-1).

While performing sampling with the lapse of time, the buffer is acidified and the temperature is reduced, to reduce the H/D exchange reaction (at a step SF-2).

The target protein is digested using the pepsin under acid conditions (at a step SF-3).

Peptide digests are extracted (at a step SF-4), and the MALDI-TOF-MS or LS/MS/MS is performed (at a step SF-5).

Fig. 10 is a flowchart of an example of fragmenting a complex of proteins A and B and measuring the fragmentation spectrum by the hydrogen-deuterium exchange method according to the present embodiment.

The proteins A and B are dissolved in the buffer or subjected to deuterium substitution separately, and leave them for a certain period of time (t1) (at a step SG-1).

The proteins A and B are mixed up to form a complex, and leave the complex for a certain period of time (t2) (at a step SG-2).

Then substitution or dilution with a water buffer is performed (at a step SG-3).

The complex is left for a certain period of time (T=t1+t2), and an H/D off exchange reaction (off change) is performed (at a step SG-4).

The fragmentation spectrum is measured by the ESI-MS (at a step SG-5).

Referring back to Fig. 3, the protein structure analyzing instrument 100 acquires the fragmentation spectrum data from the mass spectrometer 400 through the input device 112 or the network 300 by the processing performed by the fragmentation spectrum data acquiring unit 102a, and stores the fragmentation spectrum data in the fragmentation spectrum data file 106a (at a step SA-6).

The protein structure analyzing instrument 100 determines the fragment ion assignment information on the amino acid sequence of the target protein based on the measured fragmentation spectrum by the processing performed by the assignment information determining unit 102b. In addition, the protein structure analyzing instrument 100 determines the deuteration rate of each amino acid residue in the amino acid sequence of the target protein according to the measured fragmentation spectrum and the determined fragment ion assignment information by the processing performed by the deuteration rate determining unit 102j.

The deuterium rate is obtained as follows. When the assignment information determining unit 102b determines assignment information on the peptide, the deuteration rate determining unit 102j calculates a deuteration ratio by dividing a mass increase by the number of exchangeable protons for each amino acid residue. The deuteration rate determining unit 102j determines the deuteration rate of the amino acid residue in each peptide sequence based on the deuteration ratio.

The solvent accessible residue information determining unit 102d specifies a region of the amino acid sequence of the target protein which region is in contact with the solvent according to the determined deuteration rate of each amino acid residue in the amino acid sequence. In addition, the solvent accessible residue information determining unit 102d determines the solvent accessible residue information on the amino acid sequence of the target protein according to the specified region (at a step SA-7). The solvent accessible residue information determining unit 102d stores the determined solvent accessible residue information in the solvent accessible residue information / contact region information file 106 (at a step SA-8). The site having a slower deuteration rate than that measured in a control experiment in which no hydrogen-deuterium exchange reaction is provoked may also be determined as the solvent accessible residue information.

The contact region information determining unit 102e determines the contact region information on the amino acid sequence of the target protein according to the determined deuteration rate of each amino acid sequence (at the step SA-7). In addition, contact region information determining unit 102e stores the determined contact region information in the solvent accessible residue information and contact region information file 106 (at the step SA-8). The site having a lower deuteration rate than that measured when the complex is not formed may be determined as the contact region information. Alternatively, the site having a lower deuteration rate than that measured when the complex is not formed may be determined as the contact region information in a protein complex.

The protein structure analyzing instrument 100 predicts the three-dimensional structure of the target protein by the processing performed by the three-dimensional structure prediction processing unit 102f (at a step SA-9). The protein structure analyzing instrument 100 then stores the predicted three-dimensional structure prediction data in the three-dimensional structure prediction data file 106d (at a step SA-10).

The protein structure analyzing instrument 100 outputs the easily cleavable domain information, the solvent accessible residue information, and the contact region information thus determined together with the prediction data, or uses such information for determining the predicted structure evaluation information.

Namely, the processing result output unit 102h outputs the predicted three-dimensional structure prediction data and the easily cleavable domain information, solvent accessible residue information, and contact region information thus determined while making them correspond to one another (at a step SA-11).

The predicted structure evaluation information determining unit 102g compares the predicted three-dimensional structure prediction data with the determined easily cleavable domain information, solvent accessible residue information, and contact region information (at a step SA-12). The predicted structure evaluation information determining unit 102g evaluates the predicted structure region corresponding to the easily cleavable domain specified by the easily cleavable domain information, the solvent accessible residue specified by the solvent accessible residue information, or the interactive interface specified by the contact region information, thereby determining the predicted structure evaluation information (at a step SA-13).

The predicted structure evaluation information output unit 102i outputs the determined predicted structure evaluation information (at a step SA-14). The main processing is thus completed.

### Example 1

Example 1 of the present invention will be explained in detail mainly with reference to Fig. 13. Fig. 13 is a flowchart of the system of Example 1 according to the present invention.

The mass spectrometer 400 measures the fragmentation spectrum for the target protein (or complex) (at a step SJ-1). Alternatively, the mass spectrometer 400 may measure the fragmentation spectrum while provoking the hydrogen-deuterium exchange reaction for the target protein (or complex). The protein structure analyzing instrument 100 acquires the fragmentation spectrum data from the mass spectrometer 400 through the input device 112 or the network 300 by the processing performed by the fragmentation spectrum data acquiring unit 102a. The protein structure analyzing instrument 100 then stores the fragmentation spectrum data in the fragmentation spectrum data file 106a.

The protein structure analyzing instrument 100 then determines the fragment ion assignment information on the amino acid sequence of the target protein based on the measured fragmentation spectrum by the processing performed by the assignment information determining unit 102b (at a step SJ-2). In addition, the protein structure analyzing instrument 100 specifies the region of the amino acid sequence of the target protein, in which said region of the target protein is dissociated to fragment ions, by the determined fragment ion assignment information by the process of the easily cleavable domain determining unit 102c, and determines the easily cleavable domain information on the amino acid sequence of the target protein according to the specified region.

If the hydrogen-deuterium exchange reaction is provoked, the protein structure analyzing instrument 100 determines the deuteration rate of each amino acid residue in the amino acid sequence of the target protein according to the measured fragmentation spectrum and the determined fragmented ion assignment information by the processing performed by the deuteration rate determining unit 102j. The solvent accessible residue information determining unit 102d specifies the region of the amino acid sequence of the target protein which region is in contact with the solvent according to the determined deuteration rate of each amino acid residue in the amino acid sequence. In addition, the solvent accessible residue information determining unit 102d determines the solvent accessible residue information on the amino acid sequence of the target protein according to the specified region, and stores the solvent accessible residue information in the solvent accessible residue information / contact region information file 106c. The contact region information determining unit 102e determines the contact region information on the amino acid sequence of the target protein according to the determined deuteration rate of each amino acid residue in the amino acid sequence. In addition, the contact region information determining unit 102e stores the contact region information in the solvent accessible residue information / contact region information file 106c.

Furthermore, the protein structure analyzing instrument 100 conducts a homology search for the target protein using the protein database such as the PDB by the processing performed by the three-dimensional structure prediction processing unit 102f, and extracts a structure-known protein that serves a model of the three-dimensional structure (at a step SJ-3).

The three-dimensional structure prediction processing unit 102f executes an alignment between the target protein and the structure-known protein serving as the protein model having the three-dimensional structure by the scheme such as 3D-1D method (at a step SJ-4). The three-dimensional structure prediction processing unit 102f creates the three-dimensional structure prediction data that is data on the three-dimensional structure model of the target protein, and stores the created three-dimensional structure prediction data in the three-dimensional structure prediction data file 106d (at a step SJ-5).

The processing result output unit 102h outputs the predicted three-dimensional structure prediction data and the determined easily cleavable domain information, solvent accessible residue information, and contact region information while making them correspond to one another (at a step SJ-6).

The predicted structure evaluation information determining unit 102g compares the predicted three-dimensional structure prediction data with the determined easily cleavable domain information, solvent accessible residue information, and contact region information. The predicted structure evaluation information determining unit 102g evaluates the predicted structure region corresponding to the easily cleavable domain specified by the easily cleavable domain information, the solvent accessible residue specified by the solvent accessible residue information, or the interactive interface specified by the contact region information, thereby determining the predicted structure evaluation information. The predicted structure evaluation information output unit 102i outputs the determined predicted structure evaluation information (at a step SJ-7). The processing in Example 1 is thus completed.

### Example 2

Example 2 of the present invention will be explained in detail mainly with reference to Fig. 14. Fig. 14 is a flowchart of the system of Example 2 according to the present invention.

The mass spectrometer 400 measures the fragmentation spectrum for the target protein (or complex) (at a step SK-1). Alternatively, the mass spectrometer 400 may measure the fragmentation spectrum while provoking the hydrogen-deuterium exchange reaction for the target protein (or complex). The protein structure analyzing instrument 100 acquires the fragmentation spectrum data from the mass spectrometer 400 through the input device 112 or the network 300 by the processing performed by the fragmentation spectrum data acquiring unit 102a. The protein structure analyzing instrument 100 then stores the fragmentation spectrum data in the fragmentation spectrum data file 106a.

The protein structure analyzing instrument 100 then determines the fragment ion assignment information on the amino acid sequence of the target protein based on the measured fragmentation spectrum by the processing performed by the assignment information determining unit 102b (at a step SK-2). In addition, the protein structure analyzing instrument 100 specifies the region of the amino acid sequence of the target protein, in which said region of the target protein is dissociated to fragment ions, by the determined fragment ion assignment information, and determines the easily cleavable domain information on the amino acid sequence of the target protein according to the specified region.

If the hydrogen-deuterium exchange reaction is provoked, the protein structure analyzing instrument 100 determines the deuteration rate of each amino acid residue in the amino acid sequence of the target protein according to the measured fragmentation spectrum and the determined fragmented ion assignment information by the processing performed by the deuteration rate determining unit 102j. The solvent accessible residue information determining unit 102d specifies the region of the amino acid sequence of the target protein which region is in contact with the solvent according to the determined deuteration rate of each amino acid residue in the amino acid sequence. In addition, the solvent accessible residue information determining unit 102d determines the solvent accessible residue information on the amino acid sequence of the target protein according to the specified region, and stores the solvent accessible residue information in the solvent accessible residue information / contact region information file 106c. The contact region information determining unit 102e determines the contact region information on the amino acid sequence of the target protein according to the determined deuteration rate of each amino acid residue in the amino acid sequence. In addition, the contact region information determining unit 102e stores the contact region information in the solvent accessible residue information / contact region information file 106c.

Furthermore, the protein structure analyzing instrument 100 conducts a homology search for the target protein using the protein database such as the PDB by the processing performed by the three-dimensional structure prediction processing unit 102f, and extracts a structure-known protein that serves as a protein model of the three-dimensional structure (at a step SK-3).

If the protein model cannot be extracted, the three-dimensional structure prediction processing unit 102f creates three-dimensional structure prediction data that is data on the three-dimensional structure model of the target protein by the scheme such as the molecular simulation method. In addition, the three-dimensional structure prediction processing unit 102f stores the created three-dimensional structure prediction data in the three-dimensional structure prediction data file 106d (at a step SK-4).

The processing result output unit 102h outputs the predicted three-dimensional structure prediction data and the determined easily cleavable domain information, solvent accessible residue information, and contact region information while making them correspond to one another (at a step SK-5).

The predicted structure evaluation information determining unit 102g compares the predicted three-dimensional structure prediction data with the determined easily cleavable domain information, solvent accessible residue information, and contact region information. The predicted structure evaluation information determining unit 102g evaluates the predicted structure region corresponding to the easily cleavable domain specified by the easily cleavable domain information, the solvent accessible residue specified by the solvent accessible residue information, or the interactive interface specified by the contact region information, thereby determining the predicted structure evaluation information. The predicted structure evaluation information output unit 102i outputs the determined predicted structure evaluation information (at a step SK-6). The processing in Example 2 is thus completed.

### Example 3

Example 3 of the present invention will be explained in detail mainly with reference to Fig. 15. Fig. 15 is a flowchart of the system of Example 3 according to the present invention.

The mass spectrometer 400 measures the fragmentation spectrum for the target protein (or complex) (at a step SL-1). Alternatively, the mass spectrometer 400 may measure the fragmentation spectrum while provoking the hydrogen-deuterium exchange reaction for the target protein (or complex). The protein structure analyzing instrument 100 acquires the fragmentation spectrum data from the mass spectrometer 400 through the input device 112 or the network 300 by the processing performed by the fragmentation spectrum data acquiring unit 102a. The protein structure analyzing instrument 100 then stores the fragmentation spectrum data in the fragmentation spectrum data file 106a.

The protein structure analyzing instrument 100 determines the fragment ion assignment information on the amino acid sequence of the target protein based on the measured fragmentation spectrum by the processing performed by the assignment information determining unit 102b (at a step SL-2). In addition, the protein structure analyzing instrument 100 specifies the region of the amino acid sequence of the target protein, in which said region of the target protein is dissociated to fragment ions, according to the determined fragment ion assignment information by the processing performed by the easily cleavable domain information determining unit 102c, and determines the easily cleavable domain information on the amino acid sequence of the target protein according to the specified region.

If the hydrogen-deuterium exchange reaction is provoked, the protein structure analyzing instrument 100 determines the deuteration rate of each amino acid residue in the amino acid sequence of the target protein according to the measured fragmentation spectrum and the determined fragmented ion assignment information by the processing performed by the deuteration rate determining unit 102j. The solvent accessible residue information determining unit 102d specifies the region of the amino acid sequence of the target protein which region is in contact with the solvent according to the determined deuteration rate of each amino acid residue in the amino acid sequence. In addition, the solvent accessible residue information determining unit 102d determines the solvent accessible residue information on the amino acid sequence of the target protein according to the specified region, and stores the solvent accessible residue information in the solvent accessible residue information / contact region information file 106c. The contact region information determining unit 102e determines the contact region information on the amino acid sequence of the target protein according to the determined deuteration rate of each amino acid residue in the amino acid sequence. In addition, the contact region information determining unit 102e stores the contact region information in the solvent accessible residue information / contact region information file 106c.

Furthermore, the protein structure analyzing instrument 100 conducts a homology search for the target protein using the protein database such as the PDB by the processing performed by the three-dimensional structure prediction processing unit 102f, and extracts candidate proteins that are structure-known proteins to serve as protein models of the three-dimensional structure (at a step SL-3).

The three-dimensional structure prediction processing unit 102f executes an alignment between the target protein and each candidate protein by the scheme such as 3D-1 D method (at a step SL-4).

The processing result output unit 102h outputs an amino acid sequence of each candidate protein and structure data on the candidate protein as well as the determined easily cleavable domain information, solvent accessible residue information, and contact region information while making them correspond to one another (at a step SL-5).

The predicted structure evaluation information determining unit 102g compares the structure data on each candidate protein with the determined easily cleavable domain information, solvent accessible residue information, and contact region information. The predicted structure evaluation information determining unit 102g evaluates the predicted structure region corresponding to the easily cleavable domain specified by the easily cleavable domain information, the solvent accessible residue specified by the solvent accessible residue information, or the contact region specified by the contact region information in a protein complex, thereby determining the predicted structure evaluation information. The predicted structure evaluation information output unit 102i outputs the determined predicted structure evaluation information. As a result, degrees of match of pieces of structure data on the respective candidate proteins with experimental data on the target protein can be given ranks, and the candidate protein having a highest evaluation can be narrowed down (at a step SL-6).

The three-dimensional structure prediction processing unit 102f creates three-dimensional structure prediction data that is data on a three-dimensional structure model of the target protein, with the candidate protein having the highest evaluation as the protein model. In addition, the three-dimensional structure prediction processing unit 102f stores the created three-dimensional structure prediction data in the three-dimensional structure prediction data file 106d (at a step SL-7).

Similarly to the steps SJ-6 and SJ-7 in Example 1, the model structure evaluation and the like are performed (at a step SL-8). The processing in Example 3 is thus completed.

### Example 4

Example 4 of the present invention will be explained in detail mainly with reference to Fig. 16. Fig. 16 is a flowchart of the system of Example 4 according to the present invention.

The protein structure analyzing instrument 100 conducts a homology search for the target protein using the protein database such as the PDB by the processing performed by the three-dimensional structure prediction processing unit 102f, and extracts a structure-known protein that serves as a protein model of the three-dimensional structure (at a step SM-1).

If the protein model cannot be extracted, the three-dimensional structure prediction processing unit 102f creates three-dimensional structure prediction data that is data on a three-dimensional structure model of the target protein by the scheme such as the molecular simulation method. In addition, the three-dimensional structure prediction processing unit 102f stores the created three-dimensional structure prediction data in the three-dimensional structure prediction data file 106d (at steps SM-2 to SM-3).

The mass spectrometer 400 measures the fragmentation spectrum for the target protein (or complex) and the protein model (at steps SM-1 and SM-4). Alternatively, the mass spectrometer 400 may measure the fragmentation spectrum while provoking the hydrogen-deuterium exchange reaction for the target protein (or complex) and the protein model. The protein structure analyzing instrument 100 acquires the fragmentation spectrum data from the mass spectrometer 400 through the input device 112 or the network 300 by the processing performed by the fragmentation spectrum data acquiring unit 102a. The protein structure analyzing instrument 100 then stores the fragmentation spectrum data in the fragmentation spectrum data file 106a.

The protein structure analyzing instrument 100 determines the fragment ion assignment information on the amino acid sequence of the target protein and the protein model based on the measured fragmentation spectrum by the processing performed by the assignment information determining unit 102b (at a step SM-2). In addition, the protein structure analyzing instrument 100 specifies the region of the amino acid sequence of the target protein and the protein model in which region the target protein or the protein model is dissociated to fragment ions according to the determined fragment ion assignment information by the processing of the easily cleavable region information determining unit 102c, and determines the easily cleavable domain information on the amino acid sequence of the target protein according to the specified region.

If the hydrogen-deuterium exchange reaction is provoked, the protein structure analyzing instrument 100 determines the deuteration rate of each amino acid residue in the amino acid sequence of the target protein and the protein model according to the measured fragmentation spectrum and the determined fragmented ion assignment information by the processing performed by the deuteration rate determining unit 102j. The solvent accessible residue information determining unit 102d specifies the region of the amino acid sequence of the target protein and the protein model which region is in contact with the solvent according to the determined deuteration rate of each amino acid residue in the amino acid sequence. In addition, the solvent accessible residue information determining unit 102d determines the solvent accessible residue information on the amino acid sequence of the target protein according to the specified region, and stores the solvent accessible residue information in the solvent accessible residue information / contact region information in a protein complex file 106c. The contact region information in a protein complex determining unit 102e determines the contact region information in a protein complex on the amino acid sequence of the target protein and the protein model according to the determined deuteration rate of each amino acid residue in the amino acid sequence. In addition, the contact region information in a protein complex determining unit 102e stores the contact region information in a protein complex in the solvent accessible residue information / contact region information in a protein complex file 106c.

The processing result output unit 102h outputs the predicted three-dimensional structure prediction data and the determined easily cleavable domain information, solvent accessible residue information, and contact region information in a protein complex as to the target protein and the protein model, while making them correspond to one another (at a step SM-5).

The predicted structure evaluation information determining unit 102g compares the predicted three-dimensional structure prediction data with the determined easily cleavable domain information, solvent accessible residue information, and contact region information in a protein complex. The predicted structure evaluation information determining unit 102g evaluates the predicted structure region corresponding to the easily cleavable domain specified by the easily cleavable domain information, the solvent accessible residue specified by the solvent accessible residue information, or the contact region specified by the contact region information in a protein complex, thereby determining the predicted structure evaluation information. The predicted structure evaluation information output unit 102i outputs the determined predicted structure evaluation information (at a step SM-6). The processing in Example 4 is thus completed.

In Example 4, the example in which the protein model cannot be extracted by the homology search at the step SM-2 has been explained. If the protein model can be extracted by the homology search, an experimental value is acquired for the protein model at the step SM-4.

### Example 5

Example 5 of the present invention will be explained in detail mainly with reference to Figs. 11 and 18.

### (1) Preparation of recombinant human interleukin-6 (IL-6)

The IL-6 was expressed with E. coli and purified (see D. Ejima: "Biotechnol. Bioeng. 62", 301-10 (1999)).

### (2) Measurement of IL-6 by FTICR-MS

All measurements were conducted using a gas assisted dynamic trapping (GADT) method. Upon capturing the ions in a measurement cell in the GADT method, a trap voltage is temporally elevated and an argon gas is introduced into the measurement cell in a manner of pulses so as to temporarily decrease an ion kinetic energy, whereby the ions can be efficiently captured in the cell and measurement sensitivity may thus be improved.

A measuring instrument, analysis conditions, and the like are as follows.
· Mass spectrometer: 7T self shield superconductive magnet Fourier transform mass spectrometer Apex II (manufactured by Bruker Daltonics Corporation);
· Flow rate: 60 µl/h;
· Drying gas: 20 psi, 150°C;
· Nebulizing gas: 30 psi;
· Argon gas pressure: 7.8 Torr;
· Intra-hexapole accumulation time: one second;
· Capillary outlet voltage: 106.6 kV (MS spectrum), 150 kV (IRMPD), 220 kV (hexapole CID)s; and
· Number of data acquisition points: 512K.

### (3) Fragmentation of IL-6 by IRMPD method and hexapole CID method

With the IRMPD method, the ions confined in an ion cyclotron cell were irradiated with a carbon dioxide gas laser for 300 milliseconds. With the hexapole CID method, fragmentation was successfully induced by increasing the capillary outlet voltage and setting the intra-hexapole accumulation time to one to three seconds using the same pulse sequence as that used in an ordinary measurement. All FTICR-MS spectra are analyzed by an Xmass that is software attached to the instrument. Software Frag-Pro (manufactured by MS/MS Software) is used for fragment ion assignment.

### (4) Homology search by 3D-1D method and PSI-BLAST

The amino acid sequence of the human interleukin-6 (IL-6) was obtained from PIR (a protein amino acid sequence database). The number of amino acid residues including the pre-sequence was 212. As a result of the homology search by the 3D-1 D method using the LIBRA, a list of 30 highest PDB files consisting of the following items was obtained; 1alu, 1lki, 1au1_A, 1nf1_A, 1qkm_A, 1jnk, 1pme, 1dg9_A, 1b5I, 1bgc, 1yrg_A, 1euv_A, 2tps_B, 1bg0, 1ial_A, 2cnd, 1cjs_A, 1wer, 7odc_A, 1eqf_A, 1f6f_A, 1dxy, 1edt, 1gse_A, 1qr2_A, 1llp, 1qi7_A, 2bid_A, 2ljr_A, and 1c3j_A.

Furthermore, as a result of the homology search under conditions of the e-value of 0.01 or less using the PSI-BLAST, 1IL6, 1ALU, 1GNC, 1BGE_B, 1 BGC, 1RHG_C, and 1I1R_B were obtained as a PDB file list. Since the six highest files are the IL6 itself or those belong to GCSF (Granulocyte Colony Stimulating Factor) that are proteins already pointed out to be similar in three-dimensional structure to the IL6. Therefore, it was confirmed that any reference protein can be a candidate for predicting the three-dimensional structure.

### (5) Making reference to alignment for secondary structure information and MS fragmentation data

For 30 alignments obtained by the homology search using the LIBRA, a program for simultaneously making reference to the secondary structure information and MS fragmentation data as to the reference proteins was created, and a result was displayed for every alignment. Fig. 18 is one example of processing results in Example 5 ((a) is followed by (b) in Fig. 18). As shown in this figure, a processing result display screen includes a display region MB-1 for displaying the amino acid sequence of the target protein, a display region MB-2 for displaying the amino acid sequence of the protein model, a display region MB-3 for displaying secondary three-dimensional structure prediction data that is one example of the three-dimensional structure prediction data, and a display region MB-4 for displaying fragments of fragment ions as experimental data. In Fig. 18, in the display region MB-3, "1" denotes an α helix, "2" denotes a β strand, and "4" denotes a loop region as secondary structures.

### (6) Evaluation of candidate proteins

A program for evaluating (giving ranks to) candidate proteins for each alignment from the secondary structure information and all of the fragmentation data was created. As a result of verifying the program, it was confirmed that the most effective protein for the IL6 three-dimensional structure prediction was the IL6 itself (1alu), and that the second most effective candidate protein was the GCSF (1bgc) that is the protein already pointed out to be similar in three-dimensional structure to the IL6.

Fig. 11 is a flowchart of one example of analysis of the fragmentation spectrum in this Example.

The assignment information determining unit 102b refers to the fragmentation spectrum stored in the fragmentation spectrum data file 106a, and acquires m/z and the number of valences of each ion in the fragmentation spectrum (in the case of low resolution, the number of valences may be unknown) (at a step SH-1).

The assignment information determining unit 102b determines assignment of each fragment ion to the amino acid sequence, and searches, extracts, and displays candidate peptide fragments based on the m/z (+number of valences) of each ion (at a step SH-2).

The easily cleavable domain information determining unit 102c extracts easily cleavable sites and stores the extracted sites as the easily cleavable domain information in the easily cleavable domain information file 106b (at a step SH-3).

The processing result output unit 102h displays the three-dimensional structure prediction data while controlling cleaved sites to be displayed in different colors based on the easily cleavable domain information using the three-dimensional structure display software, as shown in Fig. 12 (at a step SH-4). The processing in Example 5 is thus completed.

### Example 6

Example 6 of the present invention will be explained in detail mainly with reference to Fig. 26.

### (1) Measurement and assignment of fragmentation spectrum of ubiquitin using FTICR-MS

Ubiquitin derived from bovine (code No. U6253, manufactured by Sigma Corporation) was dissolved in an aqueous solution containing 1% acetic acid and 50% methanol so as to adjust the concentration to be 5 pmol/µl, which was employed as the sample. All of measurements were conducted using the GADT method.

A measuring instrument, analysis conditions, and the like are as follows.
· Mass spectrometer: 7T self shield superconductive magnet Fourier transform mass spectrometer Apex II to which a nano electro-spray ion source is attached (manufactured by Bruker Daltonics Corporation);
· Flow rate: 200 nl/min
· Drying gas: 5 psi, 200°C;
· Intra-hexapole accumulation time: 0.6 second;
· Capillary voltage: 1800 V;
· Capillary outlet voltage: 90 V; and
· Number of data acquisition points: 512K.

In this Example, fragmentation of ubiquitin was successfully induced by using the noise skimmer CID method at a high capillary outlet voltage. A spectral analysis of the FTICR-MS spectrum and assignment of the fragmentation ions were performed in the same way as in Example 5. The amino acid sequence was obtained from the PIR.

### (2) Making reference to three-dimensional structure and MS fragmentation data

The three-dimensional structure of ubiquitin has already been known. A structure file of ubiquitin (PDB file; 1UBI.pdb) was obtained from the PDB, and the three-dimensional structure of ubiquitin was displayed as a ribbon model using three-dimensional structure display software "ViewerLite (product name)" (manufactured by Accelrys Software Inc.) as shown in Fig. 26. Fig. 26 is one example of the three-dimensional structure of the bovine-derived ubiquitin in Example 6 of the present system according to the present invention. In Fig. 26, the α helix is displayed in red, the β strand is displayed in light blue, and a dissociated site obtained by the MS is displayed in yellow. As a result, it was found out that fragmentation tends to occur at the end of the α helix and the β strand more frequently, and that fragmentation less frequently tends to occur inside the α helix and inside the β strand. The explanation of Example 6 is thus completed.

### Example 7

Example 7 of the present invention will be explained in detail mainly with reference to Fig. 27.

### (1) Measurement and assignment of fragmentation spectrum for alcohol dehydrogenase using FTICR-MS

An alcohol dehydrogenase derived from yeast (code No., manufactured by Sigma Corporation) was dissolved in an aqueous solution containing 1% acetic acid and 50% methanol so as to adjust the concentration to 5 pmol/µl, which was employed as the sample. All measurements were conducted using the GADT method.

A measuring instrument, analysis conditions, and the like are as follows.
· Mass spectrometer. 7T self shield superconductive magnet Fourier transform mass spectrometer Apex II to which a nano electro-spray ion source is attached (manufactured by Bruker Daltonics Corporation);
· Flow rate: 200 nl/min
· Drying gas: 5 psi, 200°C;
· Intra-hexapole accumulation time: one second;
· Capillary voltage: 1750 V;
· Capillary outlet voltage: 170 V; and
· Number of data acquisition points: 512K.

In this Example, fragmentation of the alcohol dehydrogenase was successfully induced by using the hexapole CID method at an increased capillary outlet voltage. A spectral analysis and a fragment assignment determination by the FTICR-MS were performed in the same way as in Example 5. The amino acid sequence was obtained from the PIR.

### (2) Modeling of alcohol dehydrogenase and making reference to MS fragmentation data

A three-dimensional structure model of the alcohol dehydrogenase was created using a fully automatic protein modeling software FAMS. The three-dimensional structure of the alcohol dehydrogenase was displayed as a ribbon model using the three-dimensional structure display software "ViewerLite (product name)" (manufactured by Accelrys Software Inc.) as shown in Fig. 27. Fig. 27 is one example of the three-dimensional structure of the alcohol dehydrogenase derived from yeast in Example 7 of the present system according to the present invention. In Fig. 27, the α helix is displayed in red, the β strand is displayed in light blue, and a dissociated site obtained by the MS is displayed in yellow. As a result, it was easily recognized visually that dissociated sites tends to occur at the end of the α helix the β strand more frequently, and tend to occur inside the α helix and inside the β strand less frequently. The model structure was well matched with the experimental data. The explanation of Example 7 is thus completed.

### Example 8; Display of three-dimensional structure of protein of interest as a result of experiment

Example 8 of the present invention will be explained in detail mainly with reference to Figs. 21 to Fig. 25.

Fig. 21 is a flowchart of Example 8 of the present system according to the present invention.

In this Example, as shown in Fig. 21, the three-dimensional data is searched and extracted, and then displayed with a three-dimensional display program such as RasMol. Such display is initiated by designating (e.g., clicking a certain point on the screen) a result of an expression analysis such as mRNA expression profiling by a DNA micro-array or the like, an identification result using the MS obtained by a proteome analysis (e.g., Mascot or SEQUEST), a result of a comparison genome analysis, a result of a protein interaction analysis (e.g., a yeast two-hybrid method or a TAP method (see Anuj Kumer: *Nature*, 415, 123-124 (2002)), and an identification number such as an accession number of a gene of interest on a metabolism pathway map or a signal transmission map, e.g., KEGG. The three-dimensional structure data to be searched may be a three-dimensional structure database (e.g., the PDB) and a model structure database (e.g., FAMSBase).

One example of presenting a correlation between an increase or a decrease in an amount of transcription and the three-dimensional structure for a genome of *Anabaena* sp. PCC7120 strain will be explained. The program in this Example is aimed at clarifying the correlation between the increase or decrease in the amount of transcription and the three-dimensional structure by linking mapped data of the amount of transcription of an mRNA transcription by the DNA micro-array with the three-dimensional structure database.

Fig. 22 is one example of a display screen that depicts the differences in expression amount by the DNA micro-array in accordance with respective colors. Each small cell on the screen denotes one gene. "Red", "blue", and "green" denote "increase", "decrease", and "no change" in the amount of transcription, respectively. Dense cells and pale cells in colors denote "presence" and "absence" of sequence homology with the three-dimensional structure database (e.g., PDB), respectively.

When a user clicks one cell that corresponds to the gene of interest, a classification number of SCOP (structural classification of proteins) and an ID (identification number) of the template PDB are shown, and click buttons (underlined part in Fig. 23) that indicates high homology PDB data (up to a fifth highest three-dimensional structure in this Example) are further displayed, as illustrated in Fig. 23.

When the user clicks the click button, an alignment display screen as shown in Fig. 24 or a three-dimensional structure display screen using the graphic software such as RasMol as shown in Fig. 25 can be displayed.

According to this Example, if the identification number (e.g., accession number) of a gene or protein of interest displayed as a result of the transcriptome analysis by the DNA micro-array, the proteome analysis (protein identification using the MS), the genome analysis (bioinformatics), or the like is clicked, the three-dimensional structure of the gene or protein can be extracted from the three-dimensional structure and structure prediction database and displayed three-dimensionally. The processing in Example 8 is thus completed.

### OTHER EMBODIMENTS

While the embodiments of the present invention have been explained so far, the invention may be carried out in a manner of a variety of other embodiments within a technical scope defined by the claims.

For example, the examples in which the protein structure analyzing instrument 100 performs processings in a standalone manner have been explained. The protein structure analyzing instrument 100 may be constituted so as to perform each processing in accordance with a request from a client terminal constituted of a separate enclosure from the protein structure analyzing instrument 100, and to transmit a result of the processing back to the client terminal.

In the examples explained above, the examples of obtaining information on the solvent accessible residue, the contact region, and the like by using the hydrogen-deuterium exchange method have been explained. However, the present invention is not limited to such an example, and a chemical modification method or the like may be used instead of the hydrogen-deuterium exchange method. For example, using a transglutaminase, a stable isotope 15N may be selectively introduced to a glutamine residue of the protein (N. Shimba, N. Yamada, K. Yokoyama, E. Suzuki, Anal. Biochem., 301, 123-127 (2002)). By combining this method with one of the embodiments explained above, a difference in solvent accessible degree for each glutamine residue can be obtained from the labeling ratio. Therefore, the information on the solvent accessible residue, the contact region, and the like may be obtainable thereby similarly to the example of using the hydrogen-deuterium exchange method.

In the aforementioned embodiments, the examples of dissociating the protein using the MS have been explained. However, the present invention is not limited to such an example. The fragment ions may be obtained using, for example, an enzyme digestion method. Namely, the protein may be fragmented by proteolysis with enzymatic activity using a digestive enzyme such as a pepsin or a trypsin.

Further, the three-dimensional structure prediction processing unit 102f may predict the three-dimensional structure using a prediction program included in the protein structure analyzing instrument 100. Alternatively, three-dimensional structure data may be created using the prediction program included in the external system 200, and the three-dimensional structure prediction data thus created may be transferred through the network 300.

Among the respective processings explained in the embodiments, all of or part of those explained to be performed automatically may be performed manually or all of or part of those explained to be performed manually may be performed automatically by any of known method.

Further, processing procedures, control procedures, specific names, information including various pieces of registration data and parameters such as search conditions, screen examples, database configurations may be arbitrarily changed unless specified otherwise.

For the protein structure analyzing instrument 100, the respective constituent elements illustrated in the drawings are only functionally conceptually and are not necessarily constituted physically as illustrated in the drawings.

For example, a part or all of the processing functions of the respective units or devices of the protein structure analyzing instrument 100 or particularly those executed by the control unit 102 can be realized by CPU (central processing unit) or a program interpreted and executed by the CPU or realized as wired logic hardware. It is noted that the program is recorded in a recording medium to be explained later and mechanically read by the protein structure analyzing instrument 100 if it is necessary to do so.

That is, a computer program for issuing an instruction to CPU in cooperation with an operating system (OS) and performing various processings is recorded in the storage unit 106 or the like that is in a form of, e.g., an ROM or an HD. This computer program is executed by being loaded on a RAM or the like, and the computer program and the CPU cooperatively constitute the control unit 102. This computer program may be recorded in an application program server connected to the protein structure analyzing instrument 100 through the arbitrary network 300, and all of or part of the computer program may be downloaded if it is necessary to do so.

A program according to the present invention may be stored in a computer readable recording medium. As used herein, this "recording medium" may include arbitrary "portable physical mediums" such as a flexible disk, a magnetooptical disk, a ROM, an EPROM, an EEPROM, a CD-ROM, an MO, and a DVD, arbitrary "fixed physical mediums" such as a ROM, a RAM, and an HD included in various computer systems, and "communication mediums" that hold the program for a short period of time, such as a communication line and a carrier wave used when the program is transmitted through a network represented by a LAN, a WAN, or the Internet.

The "program" is a data processing method described in an arbitrary language and an arbitrary description method, and a format of the program is not limited to any of a source code and a binary code. The "program" is not always constituted singularly but may be constituted to be distributed to a plurality of modules or libraries or may be those which fulfill its function in cooperation with another program which is typically the OS. In each of the apparatus shown in the embodiment, a specific configuration for reading the recording medium, reading procedures, installation procedures after reading, and the like may be well-known configuration and procedures.

Each of the various databases (the fragmentation spectrum data file 106a to the processing result data file 106f) stored in the storage unit 106 is a memory device such as a RAM or a ROM, a fixed disk device such as a hard disk, or a storage unit such as a flexible disk, or an optical disk. The databases store various programs, tables, files, databases, and webpage files used for the various processings and provision of websites.

Further, the protein structure analyzing instrument 100 may be realized by connecting a peripheral device such as a printer, a monitor, or an image scanner to an information processing apparatus such as an information processing terminal, e.g., a known personal computer or workstation, and by installing software (including programs, data, and the like) that makes the information processing apparatus realize a method according to the present invention.

Moreover, specific forms of distribution and integration of the protein structure analyzing instrument 100 are not limited to those illustrated in the drawings, and all of or part of them may be constituted so as to be distributed or integrated either functionally or physically in arbitrary units depending on loads thereof and the like. For example, each database may be constituted of an independent database apparatus independently, or a part of the processings may be realized using CGI (common gateway interface). The protein structure analyzing instrument 100 may be constituted integrally with the mass spectrometer 400.

Additionally, the network 300 may function to connect the protein structure analyzing instrument 100 to the external system 200. The network 300 may include, for example, any one of the Internet, an Intranet, a LAN (which may be wired or wireless), a VAN, a personal computer communication network, a public telephone network (which may be analog or digital), a dedicated line network (which may be analog or digital), a CATV network, a portable line exchange network and portable packet exchange network of an IMT2000, a GSM, or PDC/PDC-P, a wireless call network, a local wireless network such as Bluetooth, a PHS network, and satellite communication networks such as CS, BS, and ISDB. That is, in the present invention, the data may be transmitted and received through any arbitrary network whether the network is wired or wireless.

As explained in the above in detail, according to the present invention, ions ionized by an electro-spray ionization method or the like for the target protein whose three-dimensional structure is to be predicted, are dissociated to fragment ions by a hexapole CID method or the like, and the fragmentation spectrum is measured. The fragment ion assignment information on the amino acid sequence of the target protein is determined based on the measured fragmentation spectrum. A region of the amino acid sequence of the target protein in which region the ions are dissociated to the fragment ions is specified according to the determined fragment ion assignment information, and easily cleavable domain information on the amino acid sequence of the target protein is determined according to the specified region. The three-dimensional structure of the target protein is predicted. Predicted three-dimensional structure prediction data and the determined easily cleavable domain information are output (e.g., by graphic display or display of a list using a table) while making them correspond to each other. Such features make it possible to provide the protein structure analysis method, the protein structure analyzing instrument, the program, and the recording medium capable of performing a highly accurate prediction since the measured three-dimensional structure information is taken into consideration upon predicting the three-dimensional structure of a protein (including a gene that codes the protein) whose three-dimensional structure is to be predicted.

According to the present invention, the three-dimensional structure prediction data is graphically displayed as any one of a wire model, a ribbon model, a pipe model, a ball-and-stick model, and a space filling model, and displayed while associating the easily cleavable domain information with a corresponding displayed region of the three-dimensional structure prediction data (e.g., by link setting, display on the model, display with a specific pattern such as a shading pattern or a slant-line pattern corresponding to the easily cleavable domain information on the model). With the aforementioned features, these pieces of information can be displayed visually recognizably. The aforementioned features thus make it possible to provide the protein structure analysis method, the protein structure analyzing instrument, the program, and the recording medium capable of enabling the user to intuitively determine the reliability of the three-dimensional structure prediction data.

According to the present invention, when the three-dimensional structure prediction data is displayed by graphically or using a table, the three-dimensional structure prediction data is displayed in a different color according to its correspondence to the easily cleavable domain information. With such features, these pieces of information can be displayed visually recognizably. Such features thus make it possible to provide the protein structure analysis method, the protein structure analyzing instrument, the program, and the recording medium capable of enabling the user to intuitively determine the reliability of the three-dimensional structure prediction data.

According to the present invention, the predicted three-dimensional structure prediction data is compared with the determined easily cleavable domain information. A predicted structure region corresponding to the easily cleavable domain specified by the easily cleavable domain information is evaluated, to determine predicted structure evaluation information. The determined predicted structure evaluation information is then output. With such features, the predicted structure predicted by the calculator can be evaluated based on biochemical experiment data. Such features thus make it possible to provide the protein structure analysis method, the protein structure analyzing instrument, the program, and the recording medium capable of remarkably improving prediction accuracy.

Further, the aforementioned features make it possible to remarkably improve accuracy for predicting a function based on the three-dimensional structure upon analyzing the function of the interesting gene or protein that has been found in a genomic sequence analysis, an expression profiling analysis using a DNA chip, a proteome analysis or the like. In the genomic sequence analysis, the function is predicted in light of the three-dimensional structure of the function-unknown gene or protein, whereby the function can be estimated more efficiently. Besides, the aforementioned features make it possible to efficiently perform drug design of, e.g., an inhibitor, improvement of activity based on protein engineering, and design of a functionally modified substance.

According to the present invention, the normal vibration is calculated for the target protein, and the normal vibration-mode analysis result (e.g., flexibility information) is displayed while being associated with the corresponding displayed region of the three-dimensional structure prediction data (e.g., displayed as a vector on the three-dimensional structure model). With such a feature, the flexibility information can be taken into consideration as the easily cleavable domain. That is, such a feature makes it possible to provide the protein structure analysis method, the protein structure analyzing instrument, the program, and the recording medium capable of evaluating the prediction model by obtaining the flexibility information based on the normal vibration-mode analysis result and comparing the flexibility information with the MS measurement data.

According to the present invention, the target protein is dissociated to the fragment ions by at least one method of collisionally activated dissociation or collision induced dissociation (CID) methods that include a hexapole CID method, a nozzle skimmer CID method, a capillary skimmer CID method, an SORI-CID method, and a multi-pole store assisted capillary skimmer CID method, an IRMPD method, an in-source decay (ISD) method, a post-source decay (PSD) method, a surface-induced dissociation (SID) method, an ECD method, and a BIRD method, and the fragmentation spectrum is measured. Such features thus make it possible to provide the protein structure analysis method, the protein structure analyzing instrument, the program, and the recording medium capable of efficiently generating the fragment ions that reflect the three-dimensional structure of the target protein.

According to the present invention, the target protein is dissociated to the fragment ions by an enzyme reaction, and the fragmentation spectrum is measured. Such a feature thus makes it possible to provide the protein structure analysis method, the protein structure analyzing instruments, the program, and the recording medium capable of efficiently generating the fragment ions that reflect the three-dimensional structure of the target protein.

According to the present invention, the target protein whose three-dimensional structure is to be predicted is dissociated to fragment ions, and the fragmentation spectrum is measured. The fragment ion assignment information on the amino acid sequence of the target protein is determined based on the measured fragmentation spectrum. A region of the amino acid sequence of the target protein which region is in contact with a solvent is specified according to the measured fragmentation spectrum and the determined fragment ion assignment information, and solvent accessible residue information on the amino acid sequence of the target protein is determined according to the specified region. The three-dimensional structure of the target protein is predicted. Predicted three-dimensional structure prediction data and the determined solvent accessible residue information are output (e.g., by graphic display or display of a list using a table) while making them correspond to each other. With such features, upon predicting the three-dimensional structure of a protein (including a gene that codes the protein) the three-dimensional structure of which is unknown, the measured three-dimensional structure information may be taken into consideration. Such features thus make it possible to provide the protein structure analysis method, the protein structure analyzing instrument, the program, and the recording medium capable of performing a highly accurate prediction.

According to the present invention, the predicted three-dimensional structure prediction data is compared with the determined solvent accessible residue information. The predicted structure region corresponding to a solvent accessible residue specified by the solvent accessible residue information is evaluated, to determine the predicted structure evaluation information. The determined predicted structure evaluation information is output. With such features, the predicted structure predicted by the calculator can be evaluated based on biochemical experiment data. Such features thus make it possible to provide the protein structure analysis method, the protein structure analyzing instrument, the program, and the recording medium capable of remarkably improving prediction accuracy.

According to the present invention, after a hydrogen-deuterium exchange reaction is provoked for the target protein, the protein is dissociated and the fragmentation spectrum is measured. The deuteration rate of each amino acid residue in the amino acid sequence of the target protein is determined according to the measured fragmentation spectrum and the determined fragment ion assignment information. A region of the amino acid sequence of the target protein which region is in contact with a solvent is specified according to the deuteration rate, and solvent accessible residue information on the amino acid sequence of the target protein is determined according to the specified region. Such features thus make it possible to provide the protein structure analysis method, the protein structure analyzing instrument, the program, and the recording medium capable of efficiently obtaining the solvent accessible information.

According to the present invention, after a chemical modification is provoked for the target protein, the protein is dissociated, and the fragmentation spectrum is measured. The chemically modified region of each amino acid residue in the amino acid sequence of the target protein is determined according to the measured fragmentation spectrum and the determined fragment ion assignment information. A region of the amino acid sequence of the target protein which region is in contact with a solvent is specified according to the chemically modified region, and solvent accessible residue information on the amino acid sequence of the target protein is determined according to the specified region. Such features thus make it possible to provide the protein structure analysis method, the protein structure analyzing instrument, the program, and the recording medium capable of efficiently obtaining the solvent accessible information. According to the present invention, the complex of the target protein and the compound (e.g., a protein, a low molecular weight compound, and nucleic acid) whose three-dimensional structure is to be predicted is dissociated to fragment ions, and the fragmentation spectrum is measured. The fragment ion assignment information on both of or one of the target protein and the compound is determined based on the measured fragmentation spectrum. Contact region information in a protein complex on both of or one of the target protein and the compound is determined according to the measured fragmentation spectrum and the determined fragmentation ion assignment information. The three-dimensional structure of both of or one of the target protein and the compound is predicted. Predicted three-dimensional structure prediction data and the determined contact region information in a protein complex are output (e.g., by graphic display or display of a list using a table) while making them correspond to each other. With such features, upon predicting the three-dimensional structure of a protein (including a gene that codes the protein) the three-dimensional structure of which is unknown, the measured three-dimensional structure information may be taken into consideration. Such features thus make it possible to provide the protein structure analysis method, the protein structure analyzing instrument, the program, and the recording medium capable of performing a highly accurate prediction.

According to the present invention, the three-dimensional structure prediction data is graphically displayed (including, for example, docking simulation) as any one of a wire model, a ribbon model, a pipe model, a ball-and-stick model, and a space filling model for both of or one of the target protein and the compound, and displayed while associating the contact region information in a protein complex with a corresponding displayed region of the three-dimensional structure prediction data (e.g., by link setting, display on the model, display with a specific pattern such as a shading pattern or a slant-line pattern corresponding to the easily cleavable domain information on the model). With such features, these pieces of information can be displayed visually recognizably. Such features thus make it possible to provide the protein structure analysis method, the protein structure analyzing instrument, the program, and the recording medium capable of enabling the user to intuitively determine the reliability of the three-dimensional structure prediction data.

According to the present invention, when the three-dimensional structure prediction data is displayed by graphically or using a table, the three-dimensional structure prediction data to both of or one of the target protein and the compound is displayed in a different color according to its correspondence to the contact region information in a protein complex. With such a feature, these pieces of information can be displayed visually recognizably. Such a feature thus makes it possible to provide the protein structure analysis method, the protein structure analyzing instrument, the program, and the recording medium capable of enabling the user to intuitively determine the reliability of the three-dimensional structure prediction data.

According to the present invention, the predicted three-dimensional structure prediction data is compared with the determined easily cleavable domain information, and the predicted structure region corresponding to an contact region specified by the contact region information is evaluated, to determine the predicted structure evaluation information. The determined predicted structure evaluation information is output. With such features, the predicted structure predicted by the calculator can be evaluated based on biochemical experiment data. Such features thus make it possible to provide the protein structure analysis method, the protein structure analyzing instrument, the program, and the recording medium capable of remarkably improving prediction accuracy.

According to the present invention, after a hydrogen-deuterium exchange reaction is provoked for the complex of the target protein and the compound, the complex is dissociated, and the fragmentation spectrum is measured. The deuteration rate of both of or one of the target protein and the compound is determined according to the measured fragmentation spectrum and the determined fragment ion assignment information. The contact region is specified according to the deuteration rate, and contact region information on both of or one of the target protein and the compound is determined according to the contact region. Such features thus make it possible to provide the protein structure analysis method, the protein structure analyzing instrument, the program, and the recording medium capable of efficiently obtaining the contact region information.

According to the present invention, after a chemical modification is provoked for the complex of the target protein and the compound, the complex is dissociated, and the fragmentation spectrum is measured. The chemically modified region of both of or one of the target protein and the compound is determined according to the measured fragmentation spectrum and the determined fragment ion assignment information. The contact region is specified for both of or one of the target protein and the compound according to the chemically modified region, and contact region information in a protein complex on both of or one of the target protein and the compound is determined according to the contact region. Such features thus make it possible to provide the protein structure analysis method, the protein structure analyzing instrument, the program, and the recording medium capable of efficiently obtaining the contact region information.

According to the present invention, fragmentation of the complex of the target protein and the compound to the fragment ions is performed by an enzyme reaction, and the fragmentation spectrum is measured. Such features thus make it possible to provide the protein structure analysis method, the protein structure analyzing instrument, the program, and the recording medium capable of efficiently performing fragmentation that reflects the three-dimensional structure of the target protein.

### INDUSTRIAL APPLICABILITY

As explained, the protein structure analysis method, the protein structure analyzing instrument, the program, and the recording medium according to the present invention can complement the three-dimensional structure data on the protein obtained by the calculation scheme by the data obtained by a biochemical experiment scheme.

The protein structure analysis method, the protein structure analyzing instrument, the program, and the recording medium according to the present invention are therefore quite useful in the bioinformatics field for performing the mass spectrometry of a protein and an analysis of the three-dimensional structure of a protein.

The present invention can be carried out widely in many industrial fields, particularly fields of pharmaceuticals, foods, cosmetics, medicine, and gene expression analysis, thus being extremely useful.

## Claims

1. A protein structure analysis method comprising:
a fragmentation spectrum measuring step of dissociating a target protein whose three-dimensional structure is to be predicted, and of measuring a fragmentation spectrum thereof;
an assignment information determining step of determining fragment ion assignment information on an amino acid sequence of the target protein based on the fragmentation spectrum measured at the fragmentation spectrum measuring step;
an easily cleavable domain information determining step of
specifying a region of the amino acid sequence of the target protein, in which said region is dissociated to fragment ions, using the fragment ion assignment information determined at the assignment information determining step,
determining easily cleavable domain information on the amino acid sequence of the target protein according to the specified region;
a three-dimensional structure predicting step of predicting the three-dimensional structure of the target protein; and
a processing result output step of outputting three-dimensional structure prediction data predicted at the three-dimensional structure predicting step and the easily cleavable domain information determined at the easily cleavable domain information determining step while making them correspond to each other.

2. The protein structure analysis method according to claim 1, wherein
at the processing result output step, the three-dimensional structure prediction data is graphically displayed as any one of a wire model, a ribbon model, a pipe model, a ball-and-stick model and a space filling model, and is displayed in association with the easily cleavable domain information with a corresponding displayed region of the three-dimensional structure prediction data.

3. The protein structure analysis method according to claim 1 or 2, wherein
at the processing result output step, the three-dimensional structure prediction data is displayed in a different color according to its corresponding easily cleavable domain information.

4. The protein structure analysis method according to any one of claims 1 to 3, further comprising:
a predicted structure evaluation information determining step of
comparing the three-dimensional structure prediction data predicted at the three-dimensional structure predicting step with the easily cleavable domain information determined at the easily cleavable domain information determining step,
of evaluating a predicted structure region corresponding to an easily cleavable domain specified by the easily cleavable domain information, and
of determining predicted structure evaluation information; and
a predicted structure evaluation information output step of outputting the predicted structure evaluation information determined at the predicted structure evaluation information determining step.

5. The protein structure analysis method according to any one of claims 1 to 4, further comprising a normal vibration calculating step of calculating a normal vibration for the target protein, wherein
at the processing result output step, a calculation result obtained at the normal vibration calculating step is displayed while being associated with the corresponding displayed region of the three-dimensional structure prediction data.

6. The protein structure analysis method according to any one of claims 1 to 5, wherein
at the fragmentation spectrum measuring step, the target protein is dissociated to the fragment ions by at least one method of collisionally activated dissociation or collision induced dissociation (CID) methods that include a hexapole CID method, a nozzle skimmer CID method, a capillary skimmer CID method, an SORI-CID method, and a multi-pole store assisted capillary skimmer CID method, an IRMPD method, an in-source decay (ISD) method, a post-source decay (PSD) method, a surface-induced dissociation (SID) method, an electron capture dissociation (ECD) method, and a black-body infrared radiative dissociation (BIRD) method, and the fragmentation spectrum is measured:

7. The protein structure analysis method according to any one of claims 1 to 6, wherein
at the fragmentation spectrum measuring step, the target protein is dissociated by proteolysis with enzymatic activity, and the fragmentation spectrum is measured.

8. A protein structure analysis method, comprising:
a fragmentation spectrum measuring step of dissociating a target protein a whose three-dimensional structure is to be predicted, and of measuring a fragmentation spectrum thereof;
an assignment information determining step of determining fragment ion assignment information on an amino acid sequence of the target protein based on the fragmentation spectrum measured at the fragmentation spectrum measuring step;
a solvent accessible residue information determining step of
specifying a region of the amino acid sequence of the target protein, in which said region is in contact with a solvent based on the fragmentation spectrum measured at the fragmentation spectrum measuring step and the fragment ion assignment information determined at the assignment information determining step,
determining solvent accessible residue information on the amino acid sequence of the target protein according to the specified region;
a three-dimensional structure predicting step of predicting the three-dimensional structure of the target protein; and
a processing result output step of outputting three-dimensional structure prediction data predicted at the three-dimensional structure predicting step and the solvent accessible residue information determined at the solvent accessible residue information determining step while making them correspond to each other.

9. A protein structure analysis method, comprising:
a fragmentation spectrum measuring step of dissociating a complex of a target protein whose three-dimensional structure is to be predicted and a compound, and of measuring a fragmentation spectrum thereof;
an assignment information determining step of determining fragment ion assignment information on both of or one of the target protein and the compound based on the fragmentation spectrum measured at the fragmentation spectrum measuring step;
an contact region information determining step of determining contact region information in a protein complex on both of or one of the target protein and the compound according to the fragment spectrum measured at the fragmentation spectrum measuring step and the fragment ion assignment information determined at the assignment information determining step;
a three-dimensional structure predicting step of predicting the three-dimensional structure of both of or one of the target protein and the compound; and
a processing result output step of outputting three-dimensional structure prediction data predicted at the three-dimensional structure predicting step and the contact region information determined at the contact region information in a protein complex determining step while making them correspond to each other.

10. A protein structure analyzing instrument, comprising:
a fragmentation spectrum data acquiring unit that acquires fragmentation spectrum data from a mass spectrometer that dissociates a target protein whose three-dimensional structure is to be predicted and measures the fragmentation spectrum thereof;
an assignment information determining unit that determines fragment ion assignment information on an amino acid sequence of the target protein based on the fragmentation spectrum data acquired by the fragmentation spectrum data acquiring unit;
an easily cleavable domain information determining unit that specifies a region of the amino acid sequence of the target protein, in which said region is dissociated to fragment ions, using the fragment ion assignment information determined by the assignment information determining unit, and that determines easily cleavable domain information on the amino acid sequence of the target protein according to the specified region;
a three-dimensional structure predicting unit that predicts the three-dimensional structure of the target protein; and
a processing result output unit that outputs three-dimensional structure prediction data predicted by the three-dimensional structure predicting unit and the easily cleavable domain information determined by the easily cleavable domain information determining unit while making them correspond to each other.

11. The protein structure analyzing instrument according to claim 10, wherein
the processing result output unit graphically displays the three-dimensional structure prediction data as any one of a wire model, a ribbon model, a pipe model, a ball-and-stick model and a space filling model, and displays the easily cleavable domain information associated with a corresponding displayed region of the three-dimensional structure prediction data.

12. The protein structure analyzing instrument according to claim 10 or 11, wherein
the processing result output unit displays the three-dimensional structure prediction data in a different color according to its corresponding the easily cleavable domain information.

13. The protein structure analyzing instrument according to any one of claims 10 to 12, further comprising:
a predicted structure evaluation information determining unit of
comparing the three-dimensional structure prediction data predicted by the three-dimensional structure predicting unit with the easily cleavable domain information determined by the easily cleavable domain information determining unit,
evaluating a predicted structure region corresponding to an easily cleavable domain specified by the easily cleavable domain information, and
determining predicted structure evaluation information; and
a predicted structure evaluation information output unit of outputting the predicted structure evaluation information determined by the predicted structure evaluation information determining unit.

14. The protein structure analyzing instrument according to any one of claims 10 to 13, further comprising a normal vibration calculating unit of calculating a normal vibration for the target protein, wherein
the processing result output step displays a calculation result obtained by the normal vibration calculating unit, where the result is displayed in association with the corresponding displayed region of the three-dimensional structure prediction data.

15. The protein structure analyzing instrument according to any one of claims 10 to 14, wherein
the fragmentation spectrum data is data on the fragmentation spectrum acquired by dissociating the target protein to the fragment ions by at least one method of collisionally activated dissociation or collision induced dissociation (CID) methods that include a hexapole CID method, a nozzle skimmer CID method, a capillary skimmer CID method, an SORI-CID method and a multi-pole store assisted capillary skimmer CID method, an IRMPD method, an in-source decay (ISD) method, a post-source decay (PSD) method, a surface-induced dissociation (SID) method, an ECD method, and a BIRD method.

16. The protein structure analyzing instrument according to any one of claims 10 to 15, wherein
the fragmentation spectrum data is data on the fragmentation spectrum acquired by dissociating the target protein by an enzyme reaction.

17. A protein structure analyzing instrument, comprising:
a fragmentation spectrum data acquiring unit that acquires fragmentation spectrum data from a mass spectrometer that dissociates a target protein whose three-dimensional structure is to be predicted and measures the fragmentation spectrum thereof;
an assignment information determining unit that determines fragment ion assignment information on an amino acid sequence of the target protein based on the fragmentation spectrum data acquired by the fragmentation spectrum data acquiring unit;
a solvent accessible residue information determining unit that specifies a region of the amino acid sequence of the target protein, in which said region is in contact with a solvent, using the fragmentation spectrum measured by the fragmentation spectrum measuring unit and the fragment ion assignment information determined by the assignment information determining unit, and that determines solvent accessible residue information on the amino acid sequence of the target protein according to the specified region;
a three-dimensional structure predicting unit that predicts the three-dimensional structure of the target protein; and
a processing result output unit that outputs three-dimensional structure prediction data predicted by the three-dimensional structure predicting unit and the solvent accessible residue information determined by the solvent accessible residue information determining unit while making them correspond to each other.

18. A protein structure analyzing instrument, comprising:
a fragmentation spectrum data acquiring unit that acquires fragmentation spectrum data from a mass spectrometer that dissociates a complex of a target protein whose three-dimensional structure is to be predicted and a compound and measures a fragmentation spectrum thereof;
an assignment information determining unit that determines fragment ion assignment information on both of or one of the target protein and the compound based on the fragmentation spectrum data measured by the fragmentation spectrum data acquiring unit;
an contact region information determining unit that determines contact region information on both of or one of the target protein and the compound according to the fragmentation spectrum measured by the fragmentation spectrum measuring unit and the fragment ion assignment information determined by the assignment information determining unit;
a three-dimensional structure predicting unit that predicts the three-dimensional structure of both of or one of the target protein and the compound; and
a processing result output unit that outputs three-dimensional structure prediction data predicted by the three-dimensional structure predicting unit and the contact region information determined by the contact region information determining unit while making them correspond to each other.

19. A program that makes a computer execute a protein structure analysis method comprising:
a fragmentation spectrum data acquiring step of acquiring fragmentation spectrum data from a mass spectrometer that dissociates a target protein whose three-dimensional structure is to be predicted and measures the fragmentation spectrum;
an assignment information determining step of determining fragment ion assignment information on an amino acid sequence of the target protein based on the fragmentation spectrum data acquired at the fragmentation spectrum data acquiring step;
an easily cleavable domain information determining step of
specifying a region of the amino acid sequence of the target protein, in which said region is dissociated to fragment ions, using the fragment ion assignment information determined at the assignment information determining step, and
determining easily cleavable domain information on the amino acid sequence of the target protein according to the specified region;
a three-dimensional structure predicting step of predicting the three-dimensional structure of the target protein; and
a processing result output step of outputting three-dimensional structure prediction data predicted at the three-dimensional structure predicting step and the easily cleavable domain information determined at the easily cleavable domain information determining step while making them correspond to each other.

20. The program according to claim 19, wherein
at the processing result output step, the three-dimensional structure prediction data is graphically displayed as any one of a wire model, a ribbon model, a pipe model, a ball-and-stick model and a space filling model, and displayed in association with the easily cleavable domain information with a corresponding displayed region of the three-dimensional structure prediction data.

21. The program according to claim 19 or 20, wherein
at the processing result output step, the three-dimensional structure prediction data is displayed in a different color according to its correspondence to the easily cleavable domain information.

22. The program according to any one of claims 19 to 21, further comprising:
a predicted structure evaluation information determining step of
comparing the three-dimensional structure prediction data predicted at the three-dimensional structure predicting step with the easily cleavable domain information determined at the easily cleavable domain information determining step,
evaluating a predicted structure region corresponding to an easily cleavable domain specified by the easily cleavable domain information, and
determining predicted structure evaluation information; and
a predicted structure evaluation information output step of outputting the predicted structure evaluation information determined at the predicted structure evaluation information determining step.

23. The program according to any one of claims 19 to 22, further comprising a normal vibration calculating step of calculating a normal vibration for the target protein, wherein
at the processing result output step, a calculation result obtained at the normal vibration calculating step is displayed in association with the corresponding displayed region of the three-dimensional structure prediction data.

24. The program according to any one of claims 19 to 23, wherein
the fragmentation spectrum data is data on the fragmentation spectrum acquired by dissociating the target protein to the fragment ions by at least one method of collisionally activated dissociation or collision induced dissociation (CID) methods that include a hexapole CID method, a nozzle skimmer CID method, a capillary skimmer CID method, an SORI-CID method and a multi-pole store assisted capillary skimmer CID method, an IRMPD method, an in-source decay (ISD) method, a post-source decay (PSD) method, a surface-induced dissociation (SID) method, an ECD method, and a BIRD method.

25. The program according to any one of claims 19 to 24, wherein
the fragmentation spectrum data is data on the fragmentation spectrum acquired by dissociating the target protein by an enzyme reaction.

26. A program that makes a computer execute a protein structure analysis method, comprising:
a fragmentation spectrum data acquiring step of acquiring fragmentation spectrum data from a mass spectrometer that dissociates a target protein whose three-dimensional structure is to be predicted and measures the fragmentation spectrum;
an assignment information determining step of determining fragment ion assignment information on an amino acid sequence of the target protein based on the fragmentation spectrum data acquired at the fragmentation spectrum data acquiring step;
a solvent accessible residue information determining step of
specifying a region of the amino acid sequence of the target protein, in which region is in contact with a solvent, using the fragmentation spectrum measured at the fragmentation spectrum measuring step and the fragment ion assignment information determined at the assignment information determining step, and
determining solvent accessible residue information on the amino acid sequence of the target protein according to the specified region;
a three-dimensional structure predicting step of predicting the three-dimensional structure of the target protein; and
a processing result output step of outputting three-dimensional structure prediction data predicted at the three-dimensional structure predicting step and the solvent accessible residue information determined at the solvent accessible residue information determining step while making them correspond to each other.

27. A program that makes a computer execute a protein structure analysis method, comprising:
a fragmentation spectrum data acquiring step of acquiring fragmentation spectrum data from a mass spectrometer that dissociates a complex of a target protein whose three-dimensional structure is to be predicted and a compound and measures the fragmentation spectrum thereof;
an assignment information determining step of determining fragment ion assignment information on both of or one of the target protein and the compound based on the fragmentation spectrum data acquired at the fragmentation spectrum data acquiring step;
an contact region information determining step of determining contact region information in a protein complex on both of or one of the target protein and the compound according to the fragmentation spectrum measured at the fragmentation spectrum measuring step and the fragment ion assignment information determined at the assignment information determining step;
a three-dimensional structure predicting step of predicting the three-dimensional structure of both of or one of the target protein and the compound; and
a processing result output step of outputting three-dimensional structure prediction data predicted at the three-dimensional structure predicting step and the contact region information determined at the contact region information determining step while making them correspond to each other.
